(19) **Europäisches Patentamt / European Patent Office / Office européen des brevets**

(11) **EP 4 679 074 A1**

(12) **EUROPEAN PATENT APPLICATION**

(43) Date of publication:
14.01.2026 Bulletin 2026/03

(21) Application number: 25188159.5

(22) Date of filing: 08.07.2025

(51) International Patent Classification (IPC):
**G01N 27/327** $^{(2006.01)}$ **G01N 33/49** $^{(2006.01)}$

(52) Cooperative Patent Classification (CPC):
**G01N 27/3274; G01N 33/49**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 10.07.2024 JP 2024111189
21.05.2025 JP 2025085177

(71) Applicant: **ARKRAY, Inc.**
**Kyoto-shi, Kyoto 601-8045 (JP)**

(72) Inventors:
• **OMOTO, Kazumasa**
**Kyoto, 602-0008 (JP)**
• **SEZAKI, Akira**
**Kyoto, 602-0008 (JP)**

(74) Representative: **Mathys & Squire**
**The Shard**
**32 London Bridge Street**
**London SE1 9SG (GB)**

(54) **COMPONENT CONCENTRATION MEASUREMENT METHOD**

(57) Provided is a method for reducing the discrepancy between a measured value of component concentration in whole blood and a measured value of component concentration in plasma. An aspect of the invention relates to a method for measuring a plasma component concentration using a whole blood sample. The method includes: measuring a whole blood component concentration using a whole blood sample, using an electrochemical sensor method; computing a correction value using a whole blood component concentration measured value obtained through the measurement, a hematocrit value of the whole blood sample, and a response index of a sensor used for the measurement of the whole blood component concentration; and computing a plasma component concentration using the correction value and the whole blood component concentration measured value.

FIG. 6

**Description**

BACKGROUND OF THE INVENTION

1. Field of the Invention

The present invention relates to a method for measuring the concentration of a component in blood plasma using a whole blood sample.

2. Description of Related Art

In the field of medicine, the measurement of concentrations of biological components using electrochemical methods is widely practiced. These methods are used to measure glucose concentration in blood, in particular. Usually, glucose concentration is measured as the concentration in blood plasma. However, this requires preliminary centrifugation of whole blood to obtain blood plasma, which is a complicated operation, and therefore, it has been proposed to measure glucose concentration using whole blood and then correct it to glucose concentration in blood plasma (JP H9-318634A).

SUMMARY OF THE INVENTION

The method disclosed in JP H9-318634A uses a whole blood sample to measure a glucose concentration measured value (EP) computed by an equilibrium point method and a glucose concentration measured value (DI) computed by a first-order differentiation method, and then converts the measured values to a glucose concentration (GL) in a plasma sample using the following formula:

$$GL = EP + a \times (EP - DI) + b \cdots (1)$$

In the formula (1) above, a and b are constants.

However, the inventors of the present invention found a problem in that there may be a discrepancy between the glucose concentration obtained using the aforementioned method and a measured value (plasma value (true value)) of glucose concentration when actually measured with blood plasma. Then, in the course of extensive research, it was found that one of the causes of the discrepancy is the hematocrit value, and the discrepancy increases with the increase in the hematocrit value. It was also found that the problem of discrepancy is particularly pronounced when the glucose concentration is high.

Therefore, the present disclosure provides a method, a measurement apparatus and a program that enable the measurement of a component concentration in blood plasma using a whole blood sample regardless of the hematocrit value. The present disclosure provides a method and a program that enable the measurement or determination of a hematocrit value using a whole blood sample.

An aspect of the present disclosure relates to a method for measuring a plasma component concentration using a whole blood sample, the method including: measuring a whole blood component concentration using a whole blood sample, using an electrochemical sensor method; computing a correction value using the whole blood component concentration measured value, a hematocrit value of the whole blood sample, and a response index of a sensor; and computing a plasma component concentration using the correction value and the whole blood component concentration measured value.

Another aspect of the present disclosure relates to a method for measuring a plasma glucose concentration using a whole blood sample, the method including: obtaining a glucose concentration measured value (EP) computed by an equilibrium point method and a glucose concentration measured value (DI) computed by a first-order differentiation method, using a glucose sensor method and using a whole blood sample; obtaining a whole blood glucose concentration value (GL) using the obtained glucose concentration measured value (EP) and glucose concentration measured value (DI), using a formula (1) below; obtaining a correction value (CR) using the whole blood glucose concentration value (GL), a hematocrit value (Hct value) of the whole blood sample, a response index of a sensor, and a standard response index r, using a formula (9) below; and obtaining a plasma glucose concentration (GL2) using the whole blood glucose concentration value (GL) and the correction value (CR), using a formula (10) below:

$$GL = EP + a \times (EP - DI) + b \cdots (1)$$

wherein, in the formula (1), a is a constant of $0.5 \le a \le 3.0$, and b is a constant of $0 < b \le 10$;

CR = Hct value × correction value determination coefficient p × (standard response index r / response index) × [GL - {(Hct value × axis determination coefficient m + axis determination coefficient n) × (response index / standard response index r)}]$^2$     (9)

wherein, in the formula (9), the correction value determination coefficient p, the standard response index r, and the axis determination coefficients m and n are constants; and

$$GL2 = GL - CR \cdots (10).$$

Yet another aspect of the present disclosure relates to an apparatus for measuring a plasma component concentration using a whole blood sample, the apparatus including: a measurement unit configured to measure a whole blood component concentration in a whole blood sample using an electrochemical sensor method; and a control unit configured to calculate a plasma component concentration from a measured value obtained by the measurement unit, wherein the control unit is configured to calculate a correction value using the whole blood component concentration measured value obtained by the measurement unit, a hematocrit value of the whole blood sample, and a response index of a sensor, and to calculate a plasma component concentration using the obtained correction value and the whole blood component concentration measured value.

Yet another aspect of the present disclosure relates to a non-transitory computer-readable storage medium storing a program for causing a computer to execute processing to determine a plasma component concentration using a whole blood sample, the processing including: a correction value computation step of computing a correction value using a measured value obtained by measuring a component concentration in the whole blood sample using an electrochemical sensor method, a hematocrit value of the whole blood sample, and a response index of a sensor; and a concentration computation step of computing a plasma component concentration using the correction value and the measured value.

Yet another aspect of the present disclosure relates to a non-transitory computer-readable storage medium storing a program for causing a computer to execute processing to determine a plasma glucose concentration using a whole blood sample, the processing including: a measurement step of performing, using a glucose sensor method and using a whole blood sample, glucose concentration measurements computed by an equilibrium point method and a first-order differentiation method; a first concentration computation step of computing a whole blood glucose concentration value (GL) using a glucose concentration measured value (EP) and a glucose concentration measured value (DI) obtained by the equilibrium point method and the first-order differentiation method, respectively, in the measurement step, using a formula (1) below; a correction value computation step of computing a correction value (CR) using the whole blood glucose concentration value (GL) obtained in the first concentration computation step, a hematocrit value (Hct value) of the whole blood sample, a response index of a sensor, and a standard response index r, using a formula (9) below; and a second concentration computation step of computing a plasma glucose concentration (GL2) using the whole blood glucose concentration value (GL) and the correction value (CR), using a formula (10) below:

$$GL = EP + a \times (EP - DI) + b \cdots (1)$$

wherein, in the formula (1), a is a constant of $0.5 \leq a \leq 3.0$, and b is a constant of $0 < b \leq 10$;

CR = Hct value × correction value determination coefficient p × (standard response index r / response index) × [GL - {(Hct value × axis determination coefficient m + axis determination coefficient n) × (response index / standard response index r)}]$^2$     (9)

wherein, in the formula (9), the correction value determination coefficient p, the standard response index r, and the axis determination coefficients m and n are constants; and

$$GL2 = GL - CR \cdots (10).$$

Yet another aspect of the present disclosure relates to a method for measuring a hematocrit value using a whole blood sample, the method including: obtaining, using a glucose sensor method and using a whole blood sample, a glucose concentration measured value (EP) computed by an equilibrium point method and a glucose concentration measured value (DI) computed by a first-order differentiation method; obtaining a whole blood glucose concentration value (GL) using the glucose concentration measured value (EP) obtained by the equilibrium point method and the glucose concentration measured value (DI) obtained by the first-order differentiation method, using a formula (1) below; and obtaining a hematocrit value using the glucose concentration measured value (EP), the glucose concentration measured value (DI), and the whole blood glucose concentration value (GL), using a formula (12) below:

$$GL = EP + a \times (EP - DI) + b \cdots (1)$$

wherein, in the formula (1), a is a constant of $0.5 \le a \le 3.0$, and b is a constant of $0 < b \le 10$; and

Hematocrit value = $(EP - DI) \times \{[Hct \text{ determination coefficient } s] \times \text{response index} + [Hct \text{ determination coefficient } t]\} \times \{(GL)^{[Hct \text{ determination coefficient } u]}\}$  (12)

wherein, in the formula (12), the Hct determination coefficients s, t, and u are constants.

Yet another aspect of the present disclosure relates to a non-transitory computer-readable storage medium storing a program for causing a computer to execute processing to determine a hematocrit value using a whole blood sample, the processing including: a measurement step of performing, using a glucose sensor method and using a whole blood sample, glucose concentration measurements using an equilibrium point method and a first-order differentiation method; a concentration computation step of computing a whole blood glucose concentration value (GL) using a glucose concentration measured value (EP) and a glucose concentration measured value (DI) obtained by the equilibrium point method and the first-order differentiation method, respectively, in the measurement step, using a formula (1) below; and a hematocrit value computation step of computing a hematocrit value using the glucose concentration measured value (EP), the glucose concentration measured value (DI), and the whole blood glucose concentration value (GL), using a formula (12) below:

$$GL = EP + a \times (EP - DI) + b \cdots (1)$$

wherein, in the formula (1), a is a constant of $0.5 \le a \le 3.0$, and b is a constant of $0 < b \le 10$; and

Hematocrit value = $(EP - DI) \times \{[Hct \text{ determination coefficient } s] \times \text{response index} + [Hct \text{ determination coefficient } t]\} \times \{(GL)^{[Hct \text{ determination coefficient } u]}\}$  (12)

wherein, in the formula (12), the Hct determination coefficients s, t, and u are constants.

According to the present disclosure, a plasma component concentration can be measured from a whole blood sample regardless of the hematocrit value. According to the present disclosure, a hematocrit value can be measured from a whole blood sample.

BRIEF DESCRIPTION OF DRAWINGS

FIG. 1 is a graph showing discrepancies between plasma glucose concentration measured values and whole blood glucose concentration values, varying with differences in hematocrit values.

FIG. 2 is a graph showing discrepancies between plasma glucose measured values and whole blood glucose concentration values, varying with differences in sensor response (response index).

FIG. 3 is a graph obtained by fitting a quadratic curve approximation formula to discrepancies between plasma glucose concentration measured values and whole blood glucose concentration values.

FIG. 4 is a graph showing the relationship between hematocrit values and axis positions, expressed as a linear equation.

FIG. 5 is a graph showing the relationship between hematocrit values obtained from whole blood glucose concentration versus differences between glucose concentration measured values (EP) computed by an equilibrium point method and glucose concentration measured values (DI) computed by a first-order differentiation method, varying with differences in glucose concentration of whole blood sample.

FIG. 6 is a diagram showing an example of the appearance of a measurement apparatus according to an embodiment.

FIG. 7 is a schematic diagram showing an example of the configuration of a reaction cell of the measurement apparatus according to an embodiment.

FIG. 8 is a flowchart illustrating the flow of plasma glucose concentration measurement using whole blood sample according to an embodiment.

FIG. 9 is a flowchart illustrating the flow of plasma glucose concentration measurement using whole blood sample according to an embodiment.

FIG. 10 is a flowchart illustrating the flow of plasma glucose concentration measurement using whole blood sample according to an embodiment.

FIG. 11 is a flowchart illustrating the flow of hematocrit value measurement using whole blood sample according to an embodiment.

FIGS. 12A to 12D are graphs showing discrepancies of uncorrected glucose concentration values (GL) and corrected glucose concentrations (GL2) from plasma glucose concentration measured values, at a response index of 0.049.

FIGS. 13A to 13D are graphs showing discrepancies of uncorrected glucose concentration values (GL) and corrected glucose concentrations (GL2) from plasma glucose concentration measured values, at a response index of 0.043.

FIGS. 14A to 14D are graphs showing discrepancies of uncorrected glucose concentration values (GL) and corrected glucose concentrations (GL2) from plasma glucose concentration measured values, at a response index of 0.042.

FIGS. 15A to 15D are graphs showing discrepancies of uncorrected glucose concentration values (GL) and corrected glucose concentrations (GL2) from plasma glucose concentration measured values, at a response index of 0.038.

DETAILED DESCRIPTION OF THE INVENTION

The present disclosure relates to a method for measuring the concentration of a component in blood plasma (plasma component concentration) from a whole blood sample containing blood cells and liquid components, and this method is characterized by using a component concentration (EP) measured from the sample using an equilibrium point method and a component concentration (DI) measured from the sample using a first-order differentiation method, and performing correction using a correction formula that includes the multiplication of a term related to the hematocrit value of the sample by a term related to the response index of a sensor. The method is preferably a computer-implemented method in which at least the computation or calculation steps are performed by a computer or other processor running a program which causes the computation or calculation steps to be executed.

The term "whole blood sample" as used in the present disclosure refers to blood that has been collected from a subject and has not been treated to separate serum or plasma. The "whole blood sample" as used in the present disclosure may or may not be subjected to anticoagulation treatment or dilution treatment with a reaction buffer during the implementation of the measurement method of the present disclosure. In the present disclosure, in one or more embodiments, a whole blood sample may or may not contain serum or plasma.

The terms "whole blood component concentration" and "whole blood component concentration measured value" as used in the present disclosure refers to concentration of a component in a whole blood sample when measured in the whole blood sample. The term "whole blood glucose concentration value (GL)" as used in the present disclosure refers to glucose concentration computed by a formula (1) below.

The term "plasma component concentration" as used in the present disclosure refers to concentration of a component in blood plasma. The term "plasma glucose concentration measured value" as used in the present disclosure refer to glucose concentration in blood plasma when actually measured with blood plasma.

In one or more embodiments, the term "plasma component concentration" as used in the present disclosure refers to the concentration of the component when measured in a plasma sample, the concentration of the component corresponding to its concentration in plasma, or the concentration obtained through conversion from the value of the component concentration measured in a whole blood sample to the component concentration in plasma. The plasma component concentration as used in the present disclosure may refer to, in one or more embodiments, a plasma component concentration calculated by measuring a component concentration from a whole blood sample and correcting the obtained measured value, using the method of the present disclosure.

In one or more non-limiting embodiments, the plasma component concentration may be a plasma glucose concentration (concentration of glucose in blood plasma).

The term "sensor response" as used in the present disclosure refers to one of the performance indicators in sensors used for measurement using an electrochemical sensor method, and in one or more embodiments, it is an indicator of how accurately or rapidly the sensor responds to changes in the concentration of a target substance. The term "response index of a sensor" as used in the present disclosure refers to an indicator of the response of a sensor used for the measurement of a whole blood component concentration using the electrochemical sensor method, and, in one or more embodiments, it can be determined as described later.

The electrochemical sensor method refers to a method for measuring the concentration of a component in a biological sample, such as blood, using a sensor that converts a state into an electrical signal by utilizing a redox potential. In one or more embodiments, the electrochemical sensor method may be a glucose sensor method or the like.

In one or more embodiments, measurement of glucose concentration using the glucose sensor method can be performed using a glucose sensor, as disclosed in JP 2004-170401A. In one or more embodiments, examples of the glucose sensor include a sensor that converts a state into an electrical signal by utilizing a redox potential. In one or more embodiments, methods for measuring glucose concentration using the glucose sensor method include an equilibrium point method and a first-order differentiation method.

Glucose concentration in blood usually refers to glucose concentration in blood plasma (glucose concentration when measured with blood plasma sample); however, for convenience of measurement, the following procedure is practiced as in the method disclosed in JP H9-318634A: measurement with whole blood is performed to obtain a glucose concentration measured value (EP) computed by an equilibrium point method and a glucose concentration measured value (DI) computed by a first-order differentiation method, and these measured values are converted to a glucose concentration (GL) in plasma using a formula (1) below:

$$GL = EP + a \times (EP - DI) + b \qquad (1)$$

In the formula (1) above, a is a constant of $0.5 \leq a \leq 3.0$, and b is a constant of $0 < b \leq 10$.

The measurement of glucose concentration computed by the equilibrium point method is performed as follows. For a standard solution with a known glucose concentration, measurement is continued until the output (current value) of a hydrogen peroxide electrode becomes substantially constant (equilibrium state) after the measurement starts (i.e., after a sample is injected into a cell), and the relationship between the constant output (sensor output value in equilibrium state) and the glucose concentration is determined as a calibration curve beforehand. Subsequently, for a sample with an unknown glucose concentration, the output of the hydrogen peroxide electrode is measured, and the measurement is continued until the output similarly becomes a substantially constant value (equilibrium state). Then, the glucose concentration is determined from this constant value (sensor output value in equilibrium state) based on the calibration curve.

More specifically, the rate of the degradation reaction of glucose by glucose oxidase is proportional to the concentration of glucose in a buffer solution. However, since the amount itself of glucose degradation by the enzyme is very small, the glucose concentration hardly changes. Therefore, in a steady state, the hydrogen peroxide production rate remains constant. The equilibrium point method uses this specific correlation between the constant output value and the glucose concentration in the buffer solution as a calibration curve.

In this equilibrium point method, a sufficient time (e.g., approximately 10 seconds) is required for the output to reach a constant value after the start of measurement. It has been found that if blood cells are mixed in a liquid sample (e.g., a whole blood sample) to be measured, this time is sufficient for glucose contained in the liquid inside the blood cells to diffuse into the buffer solution, overcoming the resistance of the blood cell membrane. Thus, the glucose that results in the glucose concentration (EP) computed by the equilibrium point method includes both the glucose dissolved in the liquid component of the liquid sample and the glucose dissolved in the liquid inside the blood cells.

On the other hand, the measurement of glucose concentration computed by the first-order differentiation method is performed as follows. For a standard solution with a known glucose concentration, the relationship between the output (current value) of a hydrogen peroxide electrode after the measurement starts and the measurement time is measured, and based on this, the relationship between the maximum value of the amount of temporal change in output (the derivative of the output over time, and thus the rate of output) and the glucose concentration is determined as a calibration curve beforehand. Subsequently, for a sample with an unknown glucose concentration, the temporal change in output of the hydrogen peroxide electrode is measured, and the maximum value of the amount of temporal change is similarly measured. Then, the glucose concentration is determined from this maximum value based on the calibration curve. More specifically, when the relationship between the electrode output and time is differentiated with respect to time to obtain the amount of temporal change in output, a curve with a local maximum is obtained. The first-order differentiation method uses a specific correlation between the maximum value of the obtained curve and the glucose concentration in the buffer solution as a calibration curve.

It has been found that, in this first-order differentiation method, the time required for the amount of temporal change in output to reach its maximum after the start of measurement is only a few seconds (e.g., approximately 2 seconds).

The inventors of the present invention found that there is a discrepancy between a glucose concentration (GL) obtained using the method of JP H9-318634A and a glucose concentration measured value actually measured using blood plasma, and one of the causes of this discrepancy is the hematocrit value of the whole blood sample. In other words, it was found that the higher the hematocrit value of the sample, the greater the discrepancy between the glucose concentration value measured using blood plasma and the glucose concentration value measured using whole blood.

Glucose concentration measurement with whole blood using the glucose sensor method can be performed using a glucose sensor and a reaction cell, as disclosed in JP 2004-170401A, for example. However, the inventors of the present invention found that the response of the glucose sensor used for measurement may fluctuate and that this fluctuation is one of the causes of the aforementioned discrepancy, or in other words, the discrepancy between the blood plasma glucose concentration value obtained by measurement using blood plasma and the blood plasma glucose concentration value obtained by measurement using whole blood increases as the response of the sensor decreases.

In one or more embodiments, the glucose sensor is composed of an electrode (such as hydrogen peroxide electrode) and a glucose oxidase immobilized enzyme membrane (GOD membrane), with the GOD membrane exposed at a leading end of the electrode. The reaction cell is filled with a buffer solution, and the glucose sensor is inserted into the reaction cell so that the GOD membrane comes into contact with the buffer solution in the reaction cell. A sample (for example, blood) is introduced into the reaction cell, and hydrogen peroxide produced during the process of glucose degradation into gluconic acid in the GOD membrane is measured using the electrode. Thus, the glucose concentration in the blood can be measured.

The response of the glucose sensor can be calculated using a formula (2), which will be described later, when performing periodic calibration using an internal standard solution (such as glucose concentration: 150 mg/dL).

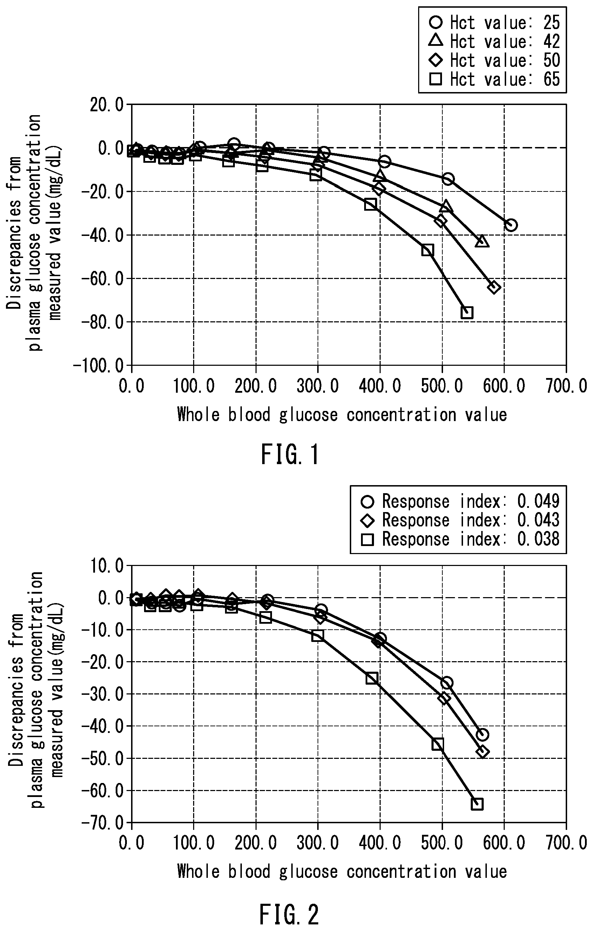

FIG. 1 is a graph showing discrepancies between plasma glucose concentration measured values and whole blood glucose concentration values computed by the formula (1) above, varying with differences in hematocrit values. It can be seen that the higher the hematocrit value and the higher the glucose concentration, the greater the discrepancy (absolute difference) from the plasma glucose concentration.

FIG. 2 is a graph showing discrepancies between plasma glucose measured values and whole blood glucose concentration values computed by the formula (1) above, varying with differences in sensor response (sensor response index). The sensor response index can be determined using a formula below, based on the maximum derivative value (DIF) of the sensor output when samples with known glucose concentrations (0 mg/dL and 150 mg/dL) are measured and the sensor output value (IV) when the sensor output reaches an equilibrium state:

$$\text{Response index } r = DIF / IV \cdots (2)$$

$$\text{where } IV = K_{STD} - K_0 \cdots (3)$$

where $K_{STD}$ represents the sensor output value (maximum A/D value) when the equilibrium state is reached during the measurement of the sample with a glucose concentration of 150 mg/dL, and

$K_0$ represents the sensor output value (maximum A/D value) when the equilibrium state is reached during the measurement of the sample with a glucose concentration of 0 mg/dL; and

$$DIF = dK_{STD} - dK_0 \cdots (4)$$

where $dK_{STD}$ represents the maximum derivative value of the sensor output (A/D value) during the measurement of the sample with a glucose concentration of 150 mg/dL, and

$dK_0$ represents the maximum derivative value of the sensor output (A/D value) during the measurement of the sample with a glucose concentration of 0 mg/dL.

In the formula (1) above, the component concentration (EP) computed by the equilibrium point method can be obtained using $K_{STD}$ and $K_0$ above, as shown in a formula (5) below, and the component concentration (DI) computed by the first-order differentiation method can be obtained using $dK_{STD}$ and $dK_0$ above, as shown in a formula (6) below:

$$EP = (K_{sample} - K_0) / (K_{STD} - K_0) \times 150 \text{ mg/dL} \cdots (5)$$

$$DI = (dK_{sample} - dK_0) / (dK_{STD} - dK_0) \times 500 \text{ mg/dL} \cdots (6)$$

where $K_{sample}$ represents the sensor output value (maximum A/D value) when the equilibrium state is reached during the measurement of a test sample, and

$dK_{sample}$ represents the maximum derivative value of the sensor output (A/D value) during the measurement of the test sample.

The inventors of the present invention noted, from FIGS. 1 and 2, that, in both cases of an increase in hematocrit value and a decrease in sensor response index, the absolute difference (discrepancy) from the plasma glucose concentration measured value (true value) changes in an upward convex parabola as the whole blood glucose concentration value increases.

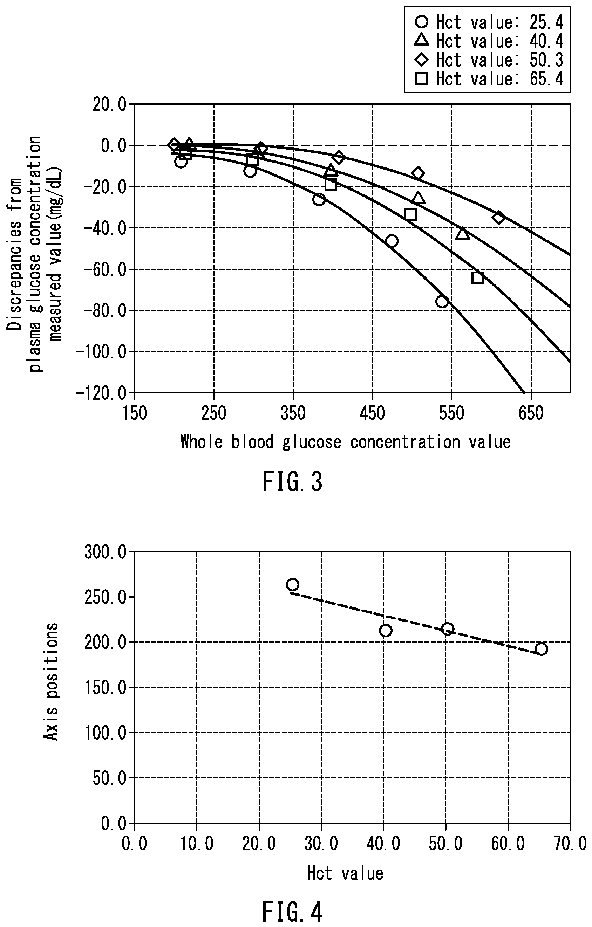

In the course of further research, the inventors of the present invention found that a graph was plotted for each hematocrit value, with the discrepancy (absolute difference) between the plasma glucose measured value and the whole blood glucose concentration value being shown on the vertical axis, and the whole blood glucose measured value being shown on the horizontal axis, as shown in FIG. 3. As a result, it was found that, when a quadratic curve approximation formula is fitted, the slope of the discrepancy (absolute difference) between the plasma measured value and the whole blood glucose concentration value varies depending on the hematocrit value; specifically, the slope increases as the hematocrit value increases (change in slope). It was also found that, when the quadratic curve approximation formula is fitted, the position of the vertex varies depending on the hematocrit value; specifically, the vertex of the quadratic curve changes in the negative direction as the hematocrit value increases (change in axis). Furthermore, it was found that similar trends are observed for the decrease in sensor response index as well, and it was found that it is preferable to apply these to a correction formula.

From these findings, the inventors of the present invention found that the discrepancy from the plasma value (true value) can be reduced by correcting the component concentration (EP) obtained by the equilibrium point method and the component concentration (DI) obtained by the first-order differentiation method for the "change in slope" and the "change in axis" using the hematocrit value in a sample and the response index of the sensor used for the measurement of the component concentrations, or specifically by performing correction using a correction formula that includes the multiplication of a term related to the hematocrit value by a term related to the sensor response index, thereby correcting a measured value (whole blood component concentration value) from a whole blood sample to a measured value (plasma component concentration measured value) from a blood plasma sample.

First, a correction method for the "change in slope" was examined.

From FIG. 3, which shows discrepancies (absolute differences) between whole blood glucose concentration values (GL) and plasma glucose concentration measured values, varying with differences in hematocrit value, the discrepancy between the whole blood glucose concentration value (GL) and the plasma glucose concentration measured value was modeled as a quadratic approximation formula. It was noted that, as shown in FIGS. 1 and 3, the slope of the quadratic approximation formula varies (increases) depending on the hematocrit value (Hct value) and increases with a predetermined coefficient (correction value determination coefficient p). Therefore, the "correction value determination coefficient p" was obtained from the slope of the approximation formula and the corresponding hematocrit value (Hct value). Furthermore, it was noted that, as shown in FIG. 2, the discrepancy (absolute difference) from the plasma glucose concentration measured value increases with the decrease in the sensor response index, and thus, a formula (7) below, which can perform correction for the slope by multiplying the obtained "correction value determination coefficient p" by [standard response index r/ response index], was derived:

$$\text{(Correction for slope)} = \text{Hct value} \times \text{correction value determination coefficient p} \times \text{(standard response index r/ response index)} \tag{7}$$

The standard response index r is the response index when measured using a standard solution with a glucose concentration of 150 mg/dL.

Next, a correction method for the "change in axis" was examined.

It was found, in FIG. 3, that the axis (position of the vertex) of the quadratic approximation formula shifts in the negative direction as the hematocrit value increases. Therefore, the hematocrit value was plotted on the horizontal axis and the position of the axis on the vertical axis, and their relationship was expressed as a linear equation in FIG. 4. By defining the slope of this linear equation as an axis determination coefficient m and its intercept as an axis determination coefficient n, a linear equation (position of axis = Hct value $\times$ m + n) that can determine the position of the axis corresponding to the hematocrit value (Hct value) was derived. Furthermore, it was noted that, as shown in FIG. 2, the discrepancy (absolute difference) from the plasma glucose concentration measured value increases with the decrease in the sensor response index, and thus, a formula (8) below, which can perform correction for the axis by multiplying the obtained equation (= Hct value $\times$ m + n) for determining the position of the axis by [response index / standard response index], was derived:

$$\text{(Correction for axis)} = \text{(Hct value} \times \text{axis determination coefficient m} + \text{axis determination coefficient n)} \times \text{(response index / standard response index r)} \tag{8}$$

Since the discrepancy (absolute difference) from the plasma glucose concentration measured value changes in a convex parabola shape, a correction formula (formula (9) below) for obtaining a correction value (CR) for correcting the whole blood glucose concentration value (GL) was derived using the above formula (7) for correction for the slope and the above formula (8) for correction for the axis.

$$\text{Correction value (CR)} = \text{Hct value} \times p \times (r/r1) \times [GL - \{(Hct \times m + n) \times (r1/r)\}]^2 \cdots (9)$$

wherein, in the formula (9),

Hct value represents the hematocrit value of a whole blood sample,
GL represents whole blood glucose concentration value,

p represents correction value determination coefficient,

r represents standard response index,

r1 represents response index, and

m and n represent axis determination coefficient. Finally, it was noted that the discrepancy (absolute difference) from the plasma glucose concentration measured value increases with the increase in the hematocrit value and the decrease in the sensor response, and thus, a formula (10) was obtained, which can achieve correction to a corrected glucose concentration (GL2), which is a plasma glucose concentration with reduced discrepancy (absolute difference) from the plasma glucose concentration measured value, by subtracting the correction value (CR) from the whole blood glucose concentration value (GL).

$$\text{Corrected glucose concentration (GL2)} = \text{whole blood glucose concentration value (GL)} - \text{correction value (CR)} \quad (10)$$

Component Concentration Measurement Method

An aspect of the present disclosure relates to a method for measuring a plasma component concentration using a whole blood sample. The method is preferably a computer-implemented method in which at least the computation or calculation steps are performed by a computer or other processor running a program which causes the computation or calculation steps to be executed. The measurement method of the present disclosure relates to a measurement method including: measuring a whole blood component concentration using an electrochemical sensor method and using a whole blood sample; calculating a correction value using a whole blood component concentration measured value obtained through the measurement, a hematocrit value of the whole blood sample, and a response index of a sensor used for the measurement of the whole blood component concentration; and calculating a plasma component concentration using the correction value and the whole blood component concentration measured value. According to the present disclosure, the concentration of a component in plasma can be measured from a whole blood sample regardless of the hematocrit value.

In one or more embodiments, the correction value can be calculated using a formula (a) below:

$$\text{Correction value} = \text{hematocrit value} \times \text{correction value determination coefficient p} \times (\text{standard response index r / response index}) \times [\text{whole blood component concentration measured value} - \{(\text{hematocrit value} \times \text{axis determination coefficient m} + \text{axis determination coefficient n}) \times (\text{response index / standard response index r)}\}]^2 \quad (a)$$

wherein, in the formula (a) above, the hematocrit value is the hematocrit value of the whole blood sample, the response index is the response index of the sensor used for the measurement of the whole blood component concentration, and the correction value determination coefficient p, the standard response index r, and the axis determination coefficients m and n are constants.

In one or more embodiments, the plasma component concentration can be calculated using a formula (b) below:

$$\text{Plasma component concentration} = \text{whole blood component concentration measured value} - \text{correction value} \quad (b)$$

wherein, in the formula (b) above, the whole blood component concentration measured value can be obtained by measuring the whole blood component concentration using an electrochemical sensor method and using a whole blood sample, and the correction value can be calculated using the formula (a) above.

In one or more embodiments, the plasma component in the method of the present disclosure may be glucose or the like in plasma. Therefore, another aspect of the present disclosure relates to a method for measuring a glucose concentration (GL2) using a whole blood sample. The term "glucose concentration (GL2)" as used in the present disclosure refers to glucose concentration in plasma.

The method for measuring a glucose concentration (GL2) of the present disclosure includes performing, using a glucose sensor method and using a whole blood sample, glucose concentration measurements using an equilibrium point method and a first-order differentiation method, thereby obtaining a glucose concentration measured value (EP) computed by the equilibrium point method and a glucose concentration measured value (DI) computed by the first-order differentiation method. The glucose concentration measurements using the equilibrium point method and the first-order differentiation method can be performed using a known method that employs a glucose sensor, and in one or more embodiments, can be performed with reference to JP H9-318634A, JP 2004-170401A, and the like.

The method for measuring a glucose concentration (GL2) of the present disclosure includes obtaining a whole blood glucose concentration value (GL) using the obtained glucose concentration measured value (EP) and glucose concentration measured value (DI), using a formula (1) below:

$$GL = EP + a \times (EP - DI) + b \cdots (1)$$

wherein, in the formula (1), a is a constant of $0.5 \leq a \leq 3.0$, and b is a constant of $0 < b \leq 10$.

In the formula (1), a and b are constants, and are determined based on the sensor used for the measurement and the measurement conditions. In one or more embodiments, a and b can be set during calibration of the sensor and/or an apparatus used for the measurement. Specifically, a and b can be obtained by performing measurements using a plurality of samples (standard solutions) with known glucose concentrations (e.g., 0 mg/dL and 150 mg/dL), obtaining a glucose concentration measured value (EP) using the equilibrium point method and a glucose concentration measured value (DI) using the first-order differentiation method, and applying the obtained values to the formula (1) above.

In one or more non-limiting embodiments, a is 0.5 or more and 3.0 or less, and b is 0 or more and 10 or less. In one or more non-limiting embodiments, a = 1.3 and b = 4.6 can also be used.

The method for measuring a glucose concentration (GL2) of the present disclosure includes obtaining a correction value (CR) using the whole blood glucose concentration value (GL) obtained using the formula (1), a hematocrit value (Hct value) of the whole blood sample, a sensor response index, and a standard response index r, using a formula (9) below:

CR = Hct value $\times$ correction value determination coefficient p $\times$ (standard response index r / response index) $\times$ [GL - {(Hct value $\times$ axis determination coefficient m + axis determination coefficient n) $\times$ (response index / standard response index r)}]$^2$     (9)

wherein, in the formula (9), the correction value determination coefficient p, the standard response index r, and the axis determination coefficients m and n are constants and, in one or more embodiments, can be obtained using a method described later.

In one or more embodiments, as the hematocrit value (Hct value) in the formulae (a) and (9), a hematocrit value that has been measured beforehand using a whole blood sample used for measurement may be used, or a hematocrit value that has been obtained using a hematocrit value measurement method of the present disclosure, which will be described later, may be used. In one or more embodiments, the hematocrit value can be measured using a commonly known microhematocrit method or an automated blood cell measurement device.

The response index in the formulae (a) and (9), which is an index with which the response of a glucose sensor used for measurement can be evaluated, can be determined through measurement during calibration using a sample (standard solution) with a known glucose concentration. In one or more embodiments, the response index can be calculated using the maximum derivative value (DIF) of the sensor output when the measurements using samples with known glucose concentrations of 0 mg/dL and 150 mg/dL are performed and the sensor output value (IV) when the sensor output reaches an equilibrium state, using the formula (2) above.

The standard response index r in the formulae (a) and (9) is a constant. The discrepancy (absolute difference) between the whole blood glucose concentration value (GL) and the plasma glucose concentration measured value varies depending on the sensor response index. Therefore, in one or more embodiments, the standard response index r serves as a reference for evaluating fluctuations of the sensor response index and can be predetermined for each sensor or apparatus. In one or more non-limiting embodiments, the response index when the glucose concentration is 150 mg/dL can be used as the standard response index r, and can be set to 0.049 in one or more non-limiting embodiments.

The correction value determination coefficient p in the formulae (a) and (9) is a constant. In one or more embodiments, the correction value determination coefficient p can be obtained by performing measurements using a plurality of samples (standard solutions) with known hematocrit values and glucose concentrations, creating a graph showing the relationship of discrepancy between the whole blood glucose concentration value (GL) and the plasma glucose concentration measured value, depending on the hematocrit value, and calculating the correction value determination coefficient p from the slope of a quadratic approximation formula derived from this graph and the hematocrit value (Hct value).

The axis determination coefficients m and n in the formulae (a) and (9) are constants. In one or more embodiments, the position of the axis corresponding to the hematocrit value is determined from a graph created in the same manner as in the calculation of the correction value determination coefficient p, a linear equation is determined, with the obtained axis position as the vertical axis and the hematocrit value as the horizontal axis, and the slope of the linear equation can be determined as the axis determination coefficient m, and its intercept can be determined as the axis determination coefficient n.

The method for measuring a glucose concentration (GL2) of the present disclosure includes obtaining a glucose concentration (GL2) using the whole blood glucose concentration value (GL) and the correction value (CR) obtained using the formula (9) above, using a formula (10) below:

$$GL2 = GL - CR \cdots (10)$$

Although the present disclosure is described using glucose concentration, it is also applicable to the measurement of the concentrations of other components, besides glucose, that can be measured using an electrochemical sensor.

In one or more embodiments, the method of the present disclosure may be implemented as a component concentration measurement program or a glucose concentration measurement program executable by a processor.

Program

Yet another aspect of the present disclosure relates to a program for determining a plasma component concentration using a whole blood sample. The program of the present disclosure is a program for causing a processor to execute a correction value calculation step of calculating a correction value using a measured value obtained through measurement of a whole blood component concentration using an electrochemical sensor method and using a whole blood sample, a hematocrit value of the whole blood sample, and a response index of a sensor used for the measurement of the whole blood component concentration; and a concentration calculation step of calculating a plasma component concentration using the correction value and the measured value.

Yet another aspect of the present disclosure relates to a program for determining a plasma glucose concentration using a whole blood sample. The program according to the present aspect is a program for causing a processor to execute a measurement step of performing, using a glucose sensor method and using a whole blood sample, glucose concentration measurements using an equilibrium point method and a first-order differentiation method; a first concentration calculation step of calculating a whole blood glucose concentration value (GL) using a glucose concentration measured value (EP) and a glucose concentration measured value (DI) obtained by the equilibrium point method and the first-order differentiation method, respectively, in the measurement step, using a formula (1) below; a correction value calculation step of calculating a correction value (CR) using the whole blood glucose concentration value (GL) obtained in the first concentration calculation step, a hematocrit value (Hct value) of the whole blood sample, a sensor response index, and a standard response index r, using a formula (9) below; and a second concentration calculation step of calculating a plasma glucose concentration (GL2) using the whole blood glucose concentration value (GL) and the correction value (CR), using a formula (10) below:

$$GL = EP + a \times (EP - DI) + b \cdots (1)$$

wherein, in the formula (1), a is a constant of $0.5 \leq a \leq 3.0$, and b is a constant of $0 < b \leq 10$;

CR = Hct value $\times$ correction value determination coefficient p $\times$ (standard response index r / response index) $\times$ [GL - {(Hct value $\times$ axis determination coefficient m + axis determination coefficient n) $\times$ (response index / standard response index r)}]$^2$ (9)

wherein, in the formula (9), the correction value determination coefficient p, the standard response index r, and the axis determination coefficients m and n are constants; and

$$GL2 = GL - CR \cdots (10)$$

In one or more embodiments, the program of the present disclosure can be supplied to a system or device via a network or a storage medium and executed by a processor in the system or device by reading and executing the program. In one or more embodiments, examples of the processor include a CPU (central processing unit), an MPU (micro processing unit), a GPU (graphics processing unit), a microprocessor, a processor core, a multiprocessor, an ASIC (application-specific integrated circuit), and an FPGA (field programmable gate array). In one or more embodiments, the program may be executed by logic circuits (hardware), dedicated circuits, and the like, formed in an IC (integrated circuit) chip, an LSI (large scale integration), and the like.

Measurement Apparatus

Yet another aspect of the present disclosure relates to an apparatus for measuring a plasma component concentration using a whole blood sample. The apparatus of the present disclosure relates to a measurement apparatus including: a measurement unit that measures a component concentration in a whole blood sample using an electrochemical sensor method; and a control unit that calculates a plasma component concentration from a measured value obtained by the measurement unit, wherein the control unit calculates a correction value using the whole blood component concentration measured value obtained by the measurement unit, a hematocrit value of the whole blood sample, and a response index of a sensor used for the measurement of the whole blood component concentration by the measurement unit and calculates a plasma component concentration using the obtained correction value and the whole blood component concentration measured value. **In** one or more embodiments, the measurement apparatus of the present disclosure can measure a plasma component concentration from a whole blood sample using the measurement method of the present disclosure.

**In** one or more embodiments, the measurement unit has at least an electrochemical sensor with an electrode for measurement and a reaction cell that can be filled with a buffer solution and into which the electrochemical sensor can be inserted. **In** one or more embodiments, the electrochemical sensor may be a glucose sensor. **In** one or more embodiments, the measurement unit may have a voltage source or the like to apply a predetermined voltage to the glucose sensor.

**In** one or more embodiments, the measurement unit can perform, using a glucose sensor method and using a whole blood sample, glucose concentration measurements using an equilibrium point method and a first-order differentiation method, thereby obtaining a glucose concentration measured value (EP) computed by the equilibrium point method and a glucose concentration measured value (DI) computed by the first-order differentiation method.

**In** one or more embodiments, the control unit may calculate a whole blood glucose concentration value (GL) using the glucose concentration measured value (EP) obtained by the equilibrium point method and the glucose concentration measured value (DI) obtained by the first-order differentiation method, using a formula (1) below:

$$GL = EP + a \times (EP - DI) + b \cdots (1)$$

wherein, in the formula (1), a is a constant of $0.5 \leq a \leq 3.0$, and b is a constant of $0 < b \leq 10$.

In one or more embodiments, the control unit may calculate a correction value (CR) using the whole blood glucose concentration value (GL), a hematocrit value (Hct value) of the whole blood sample, and a sensor response index, using a formula (a) or (9) below:

Correction value (CR) = Hct value $\times$ correction value determination coefficient p $\times$ (standard response index r / response index) $\times$ [whole blood component concentration measured value - {(Hct value $\times$ axis determination coefficient m + axis determination coefficient n) $\times$ (response index / standard response index r)}]$^2$ (a)

Correction value (CR) = Hct value $\times$ correction value determination coefficient p $\times$ (standard response index r / response index) $\times$ [GL - {(Hct value $\times$ axis determination coefficient m + axis determination coefficient n) $\times$ (response index / standard response index r)}]$^2$ (9)

wherein, in the formulae (a) and (9), the Hct value is the hematocrit value of the whole blood sample, the response index is the response index of the sensor used for the measurement of the whole blood component concentration or the whole blood glucose concentration value (GL), and the correction value determination coefficient p, the standard response index r, and the axis determination coefficients m and n are constants.

In one or more embodiments, the control unit may calculate a plasma component concentration or a plasma glucose concentration (GL2) using a formula (b) or (10) below:

Plasma component concentration = whole blood component concentration measured value - correction value (CR) (b)

$$GL2 = GL - CR \cdots (10)$$

In one or more embodiments, the measurement apparatus of the present disclosure includes a storage unit. In one or more embodiments, the storage unit can record the measurement method of the present disclosure and/or store the program of the present disclosure. In one or more embodiments, the correction value determination coefficient p, the standard response index r, and the axis determination coefficients m and n may be recorded in the storage unit.

Calculation of Hematocrit Value

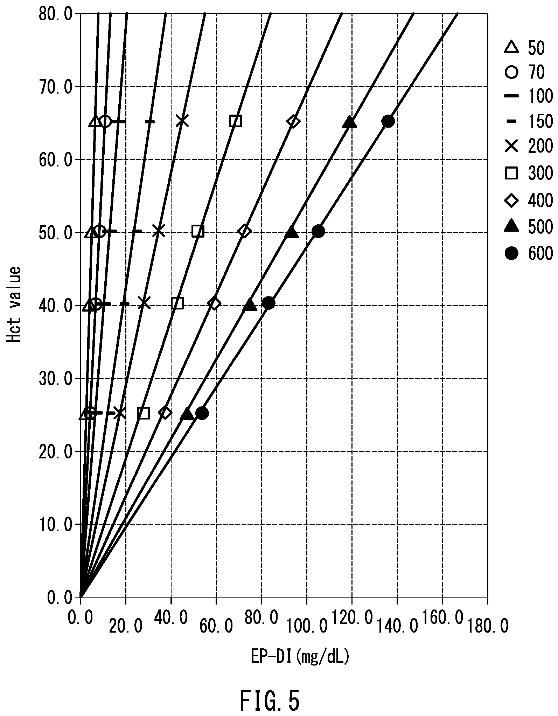

The inventors of the present invention further noted the relationship between the hematocrit value and the difference (EP - DI) between the glucose concentration measured value (EP) obtained by the equilibrium point method and the glucose concentration measured value (DI) obtained by the first-order differentiation method. FIG. 5 is a graph showing the relationship between the hematocrit value and the difference (EP - DI) between the glucose concentration measured value (EP) obtained by the equilibrium point method and the glucose concentration measured value (DI) obtained by the first-order differentiation method, with these values being obtained using a whole blood sample. The inventors of the present invention found that, as shown in FIG. 5, the difference (EP - DI) between the glucose concentration measured value (EP) obtained by the equilibrium point method and the glucose concentration measured value (DI) obtained by the first-order differentiation method increases as the hematocrit value increases. The inventors of the present invention further found that, as shown in FIG. 5, the hematocrit value and the difference (EP - DI) between the glucose concentration measured value (EP) obtained by the equilibrium point method and the glucose concentration measured value (DI) obtained by the first-order differentiation method can be approximated with a linear equation, corresponding to each glucose concentration value (GL) obtained using a whole blood sample, and the higher the glucose concentration, the more gradual the slope β of the linear equation.

A graph showing the relationship between the resulting slope β and the whole blood glucose concentration value (GL) (vertical axis: slope β, horizontal axis: GL) was created, corresponding to each sensor response index. As a result, with respect to the slope β and the whole blood glucose concentration value (GL), a power approximate formula was derived as a formula (11) below:

$$\beta = S \times [GL]^U \cdots (11)$$

Thus, it was found that the slope β can be obtained from the whole blood glucose concentration value (GL). Furthermore, it was found that the coefficient U of the power approximation formula is almost constant, independently of the sensor response index, while the coefficient S varies depending on the sensor response index.

Furthermore, using the sensor response index and the coefficient S, which corresponds to the sensor response index, an approximate straight line was created, and coefficients to be used to calculate the hematocrit value were derived from the obtained approximate straight line. Specifically, the slope of the approximate straight line was defined as "Hct determ ination coefficient, slope s", and its intercept was defined as "Hct determ ination coefficient, intercept t".

As described above, "EP - DI" increases in proportion to the hematocrit value, its slope β is a power of the whole blood glucose concentration value (GL) as shown in the formula (11) above, and the coefficient S depends on the sensor response index. Based on these findings, the inventors of the present invention derived a formula (12) below, which can calculate a hematocrit value (Hct value) using the glucose concentration measured value (EP) obtained by the equilibrium point method and the glucose concentration measured value (DI) obtained by the first-order differentiation method, which are obtained by measuring a whole blood sample using the glucose sensor method, as well as the sensor response index:

Hct value = (EP - DI) × {[Hct determination coefficient s] × response index + [Hct determination coefficient t]} × {(GL)$^{[\text{Hct determination coefficient u}]}$}　　　(12)

Conventionally, hematocrit values have been obtained by centrifuging a whole blood sample separately and measuring the height of a red blood cell layer or by directly measuring the volume of red blood cells. However, by using the formula (12) above, it is possible, in one or more embodiments, to calculate the hematocrit value (Hct value) using measured values obtained during glucose concentration measurements, without the need to centrifuge a whole blood sample or measure the volume of red blood cells.

Yet another aspect of the present disclosure relates to a method for measuring a hematocrit value using a whole blood sample. The method is preferably a computer-implemented method in which at least the computation or calculation steps are performed by a computer or other processor running a program which causes the computation or calculation steps to be executed. The method for measuring a hematocrit value of the present disclosure includes: obtaining, using a glucose sensor method and using a whole blood sample, a glucose concentration measured value (EP) computed by an equilibrium point method and a glucose concentration measured value (DI) computed by a first-order differentiation method; obtaining a whole blood glucose concentration value (GL) using the glucose concentration measured value (EP) obtained by the equilibrium point method and the glucose concentration measured value (DI) obtained by the first-order differentiation method, using a formula (1) below; and obtaining a hematocrit value using the glucose concentration measured value (EP), the glucose concentration measured value (DI), and the whole blood glucose concentration value (GL), using a formula (12) below:

$$GL = EP + a \times (EP - DI) + b \cdots (1)$$

wherein, in the formula (1), a is a constant of $0.5 \leq a \leq 3.0$, and b is a constant of $0 < b \leq 10$; and

Hematocrit value = (EP - DI) × {[Hct determination coefficient s] × response index+ [Hct determination coefficient t]} × {(GL)[Hct determination coefficient u]}   (12)

wherein, in the formula (12), the Hct determination coefficients s, t, and u are constants and can be determined using the above-described method.

Yet another aspect of the present disclosure relates to a program for determining a hematocrit value using a whole blood sample, the program causing a processor to execute a measurement step of performing, using a glucose sensor method and using a whole blood sample, glucose concentration measurements using an equilibrium point method and a first-order differentiation method; a first concentration calculation step of calculating a whole blood glucose concentration value (GL) using the glucose concentration measured value (EP) computed by the equilibrium point method and the glucose concentration measured value (DI) computed the first-order differentiation method, respectively, in the measurement step, using a formula (1) below; and a hematocrit value calculation step of calculating a hematocrit value using the glucose concentration measured value (EP), the glucose concentration measured value (DI), and the whole blood glucose concentration value (GL), using a formula (12) below:

$$GL = EP + a \times (EP - DI) + b \cdots (1)$$

wherein, in the formula (1), a is a constant of $0.5 \leq a \leq 3.0$, and b is a constant of $0 < b \leq 10$; and

Hematocrit value = (EP - DI) × {[Hct determination coefficient s] × response index + [Hct determination coefficient t]} × {(GL)[Hct determination coefficient u]}   (12)

wherein, in the formula (12), the Hct determination coefficients s, t, and u are constants and can be determined using the above-described method.

Thus, a hematocrit value can be determined using the formula (12). This hematocrit value may be used to calculate the correction value (CR).

Hereinafter, an embodiment of the method of the present disclosure will be described with reference to the drawings.

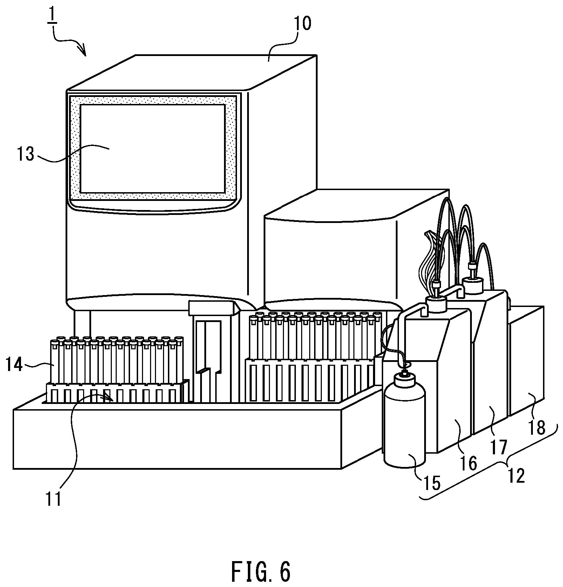

FIG. 6 shows an example of the appearance of a measurement apparatus according to the present embodiment. In the present embodiment, a case where plasma glucose concentration is measured using a whole blood sample is described as an example. The method of the present disclosure is not limited to this and can be applied to the measurement of other plasma components besides glucose.

As shown in FIG. 6, a measurement apparatus 1 is constituted by a main body portion 10, a sample supply portion 11, and a bottle unit 12. A panel 13 is provided on an upper front surface of the main body portion 10. The panel 13 is capable of operating the apparatus and displaying analyzed measured values. The sample supply portion 11 is provided at the lower front of the main body portion 10, and a plurality of sample containers 14 filled with samples (whole blood samples) can be placed therein. The bottle unit 12 is constituted by a standard solution container 15 filled with an internal standard solution, a buffer solution container 16 filled with a buffer solution, a washing solution container 17 filled with a washing solution, a drain container 18 capable of containing waste liquid discharged from the measurement apparatus 1, and the like. The containers 15, 16, 17, and 18 are connected to the main body portion 10 via tubes, respectively.

A reaction cell to which a glucose sensor can be attached is built in the main body portion 10, and the glucose sensor and the reaction cell can constitute at least a measurement unit of the measurement apparatus 1.

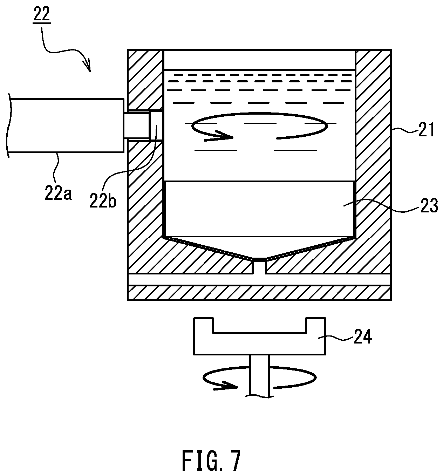

FIG. 7 shows an example of the configuration of a reaction cell 21 to which a glucose sensor 22 is attached. A stir bar 23 is disposed inside the reaction cell 21, and the buffer solution can be introduced into the reaction cell 21 through a flow path at the bottom.

A sample container 14 filled with a sample (whole blood sample) is placed in the sample supply portion 11, and the sample (whole blood sample) in the sample container 14 is drawn by a nozzle (not shown) attached to the main body portion 10 and supplied into the reaction cell 21 from the top of the reaction cell 21. The stir bar 23 can be rotated by a stirrer 24 disposed below the reaction cell 21, thereby stirring the sample and the buffer solution in the reaction cell 21.

As shown in FIG. 7, the glucose sensor 22 includes an electrode 22a, and a glucose oxidase immobilized enzyme membrane (GOD membrane) 22b is attached to a leading end of the electrode 22a. A hole for attaching the glucose sensor 22 is formed in a side face of the reaction cell 21. The GOD membrane 22b is inserted into the reaction cell 21 from the side face through this hole, and the concentration of glucose in the sample (whole blood sample) is measured in that state.

Hereinafter, an embodiment of plasma glucose concentration measurement using a whole blood sample will be described.

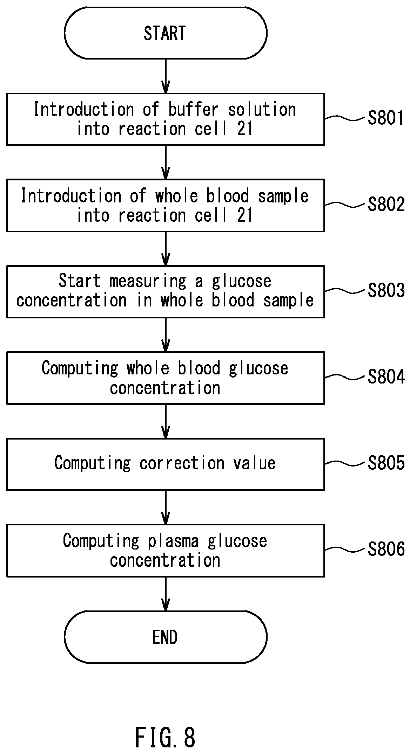

FIGS. 8 to 10 are flowcharts for describing an embodiment of plasma glucose concentration measurement using the measurement apparatus 1 shown in FIG. 6. A control unit of the measurement apparatus 1 controls the measurement by the measurement unit according to a measurement flow shown in FIGS. 8 to 10 and executes processing and the like using the obtained measured values. The control unit reads information and the like necessary for the processing and the like from a storage unit, as necessary.

First, the glucose sensor 22 is attached to the reaction cell 21 of the measurement apparatus 1, and calibration of the measurement apparatus 1 (glucose sensor 22) is performed. Calibration can be performed by filling the reaction cell 21 with the buffer solution from the buffer solution container 16 through the tube by driving a buffer solution supply pump (not shown), then supplying the standard solution into the reaction cell 21 from the standard solution container 15 through the tube by driving a standard solution supply pump (not shown), and measuring a glucose concentration in the standard solution. The constants a and b in the formula (1) above and the response indexes in the formulae (a) and (9) can be determined by performing calibration. The thus determined constants a and b and response indexes can be stored in the storage unit of the measurement apparatus 1.

Calibration of the measurement apparatus 1 does not need to be performed for each sample measurement, and can be performed at the timing when measurements have been performed a predetermined number of times, or when a new glucose sensor 22 is attached to the reaction cell 21, or the like.

Next, a plurality of sample containers 14 filled with whole blood samples are placed in the sample supply portion 11. The reaction cell 21 is filled with the buffer solution from the buffer solution container 16 through the tube by driving the buffer solution supply pump (not shown) (S801). The nozzle (not shown) is advanced into a sample container 14 by driving the nozzle, and then the whole blood sample is drawn into the nozzle by driving a sample pump (not shown). The whole blood sample drawn into the nozzle is then introduced into the reaction cell 21 through a tube (S802). Once the whole blood sample is supplied into the reaction cell 21, measurement of glucose concentration by the measurement unit is started (S803), and a glucose concentration (whole blood glucose concentration) in the whole blood sample is acquired (S804).

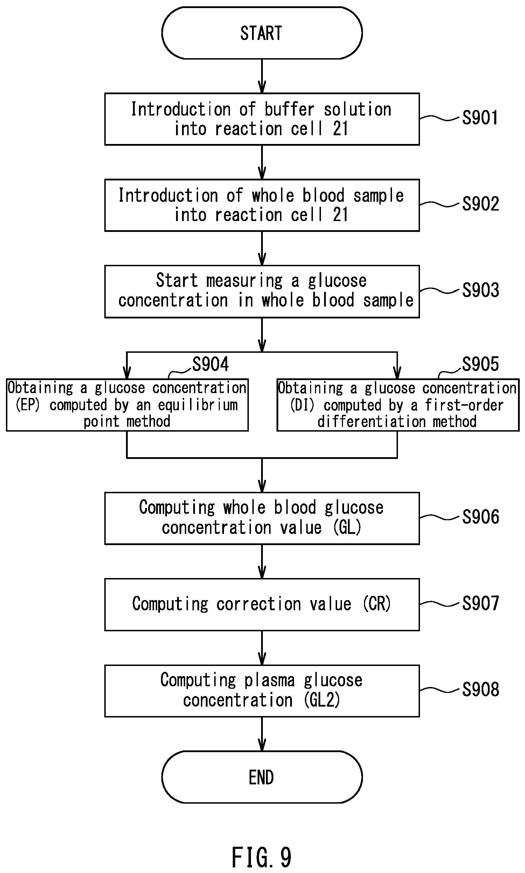

As shown in FIG. 9, the acquisition of the whole blood glucose concentration (S804) may be performed by acquiring a glucose concentration measured value (EP) using the equilibrium point method (S904) and a glucose concentration measured value (DI) using the first-order differentiation method (S905), and then acquiring a whole blood glucose concentration value (GL) using these measured values and the constants a and b obtained by the above-described calibration, using the formula (1) below (S906):

$$GL = EP + a \times (EP - DI) + b \cdots (1)$$

Next, a correction value is acquired (S805). The acquisition of the correction value (S805) can be performed using the whole blood glucose concentration value (GL), a hematocrit value of the whole blood sample, and the response indexes obtained by the above-described calibration. As the hematocrit value of the whole blood sample, a preliminarily measured value or a value acquired using the whole blood glucose concentration value (GL) as described later may be used.

As shown in FIG. 9, the acquisition of the correction value (S805) may also be performed by acquiring a correction value (CR) from the formula (9) below, using the whole blood glucose concentration value (GL), the hematocrit value (Hct value) of the whole blood sample, the response indexes, and the like (S907):

CR = Hct value $\times$ correction value determination coefficient p $\times$ (standard response index r / response index) $\times$ [GL - {(Hct value $\times$ axis determination coefficient m + axis determination coefficient n) $\times$ (response index / standard response index r)}]$^2$     (9)

In the formula (9) above, as the correction value determination coefficient p, the standard response index r, and the axis determination coefficients m and n, values recorded in the storage unit in advance or values obtained by the above-described calibration may be used.

Then, a plasma glucose concentration is acquired (S806). The acquisition of plasma glucose concentration (S806) can be performed using the whole blood glucose concentration value (GL) acquired in S804 and the correction value (CR) acquired in S805.

As shown in FIG. 9, the acquisition of the plasma glucose concentration (S806) may also be performed by acquiring a plasma glucose concentration (GL2) from the formula (10) below, using the whole blood glucose concentration value (GL) and the correction value (CR) (S908):

$$GL2 = GL - CR \cdots (10)$$

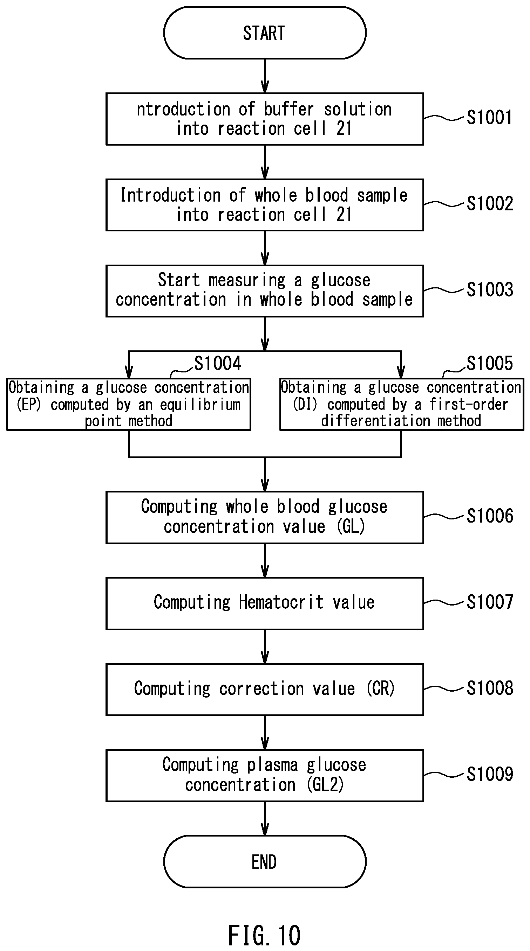

As shown in FIG. 10, the hematocrit value of the whole blood sample used in the acquisition of the correction value (S805) may also be acquired from the formula (12) below using the glucose concentration measured value (EP) computed by the equilibrium point method, acquired in S1004, and the glucose concentration measured value (DI) computed by the first-order differentiation method, acquired in S1005, as well as the whole blood glucose concentration value (GL) acquired in S1006 (S1007):

Hematocrit value = (EP - DI) × {[Hct determination coefficient s] × response index + [Hct determination coefficient t]} × {(GL)$^{[Hct\ determination\ coefficient\ u]}$}    (12)

In the formula (12) above, as the Hct determination coefficients s, t, and u, values recorded in the storage unit in advance or values obtained by the above-described calibration may be used.

Acquisition of the correction value (CR) (S1008) and acquisition of the plasma glucose concentration (GL2) (S1009) may be performed using the hematocrit value acquired in S1007.

Hereinafter, a case where the hematocrit value is measured using a whole blood sample used for plasma glucose concentration measurement, separately from or in parallel with the plasma glucose concentration measurement using the whole blood sample, will be described as an example.

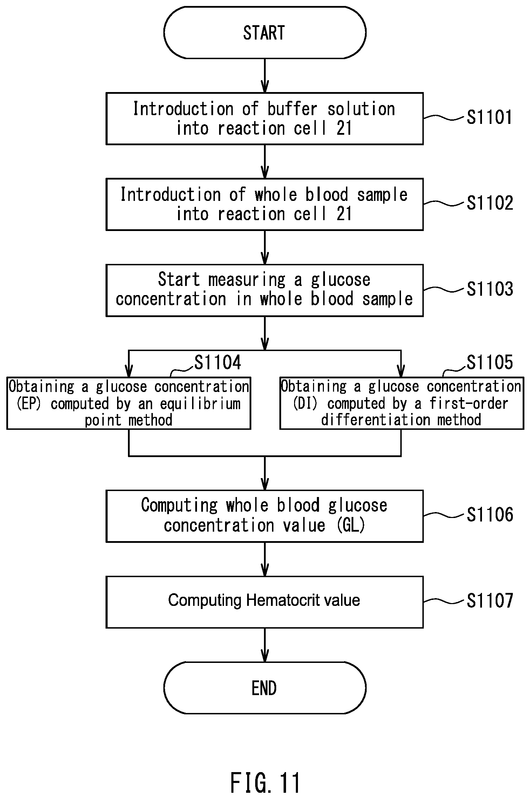

FIG. 11 is a flowchart for describing an embodiment of hematocrit value measurement using the measurement apparatus 1 shown in FIG. 6.

Calibration of the measurement apparatus 1 can be performed in the same manner as described above for the plasma glucose concentration measurement.

A plurality of sample containers 14 filled with whole blood samples are placed in the sample supply portion 11. The reaction cell 21 is filled with the buffer solution from the buffer solution container 16 through the tube by driving the buffer solution supply pump (not shown) (S1101). The nozzle (not shown) is advanced into a sample container 14 by driving the nozzle, and then the whole blood sample is drawn into the nozzle by driving the sample pump (not shown). The whole blood sample drawn into the nozzle is then introduced into the reaction cell 21 through the tube (S1102). Once the whole blood sample is supplied into the reaction cell 21, measurement of glucose concentration by the measurement unit is started (S1103), and acquisition of a glucose concentration measured value (EP) computed by the equilibrium point method (S1104) and acquisition of a glucose concentration measured value (DI) computed by the first-order differentiation method (S1105) are performed.

Using the thus obtained EP and DI as well as the constants a and b obtained by the above-described calibration, a whole blood glucose concentration value (GL) is acquired from the formula (1) below (S1106):

$$GL = EP + a \times (EP - DI) + b \cdots (1)$$

Then, a hematocrit value is acquired from the formula (12) below using EP, DI, and GL (S1107):

Hematocrit value = (EP - DI) × {[Hct determination coefficient s] × response index + [Hct determination coefficient t]} × {(GL)$^{[Hct\ determination\ coefficient\ u]}$}    (12)

In the formula (12) above, as the Hct determination coefficients s, t, and u, values recorded in the storage unit in advance or values obtained by the above-described calibration may be used.

The contents of the documents described herein are incorporated by reference as part of the present disclosure.

EXAMPLES

Samples with various glucose concentrations and hematocrit values were provided. A sample with a glucose concentration of 0 mg/dL was prepared by allowing a whole blood sample to stand at normal temperature, causing glycolysis to proceed. Glucose solutions were added to the sample with a glucose concentration of 0 mg/dL to prepare samples with glucose concentrations of 10 mg/dL, 30 mg/dL, 50 mg/dL, 70 mg/dL, 100 mg/dL, 150 mg/dL, 200 mg/dL, 300 mg/dL, 400 mg/dL, 500 mg/dL, and 600 mg/dL. From each sample, plasma was increased or reduced to obtain samples with hematocrit values adjusted to 25%, 40%, 50%, and 65%.

Four glucose analyzers, ADAMS Glucose GA-1180 manufactured by ARKRAY, Inc., were used as analyzers. The samples were subjected to measurement in a whole blood analysis mode of ADAMS Glucose GA-1180, and whole blood glucose concentration values (GL) were obtained using the formula (1) above.

Prior to the measurement, the apparatuses (glucose sensors) were calibrated, and the response indexes of the individual apparatuses were determined based on the sensor output, using the formulae (2) to (4).

Table 1

| Apparatus | Response index |
|---|---|
| Apparatus No. 1 | 0.049 |
| Apparatus No. 2 | 0.043 |
| Apparatus No. 3 | 0.038 |
| Apparatus No. 4 | 0.042 |

Glucose concentration measurement was performed using the samples with the adjusted glucose concentrations and hematocrit values, and corrected glucose concentration values (plasma glucose concentrations) (GL2) were obtained by correcting the obtained glucose concentration values (GL), which were measured values from the whole blood samples, using the formula (9) above and the parameters shown below.

Table 2

| Parameter | Numerical value |
|---|---|
| Hct coefficient (slope) [s] | -3906.558 |
| Hct coefficient (intercept) [t] | 768.370 |
| Hct coefficient (multiplier) [u] | -1.11 |
| Correction value coefficient [p] | -0.00000876 |
| Standard response index [r] | 0.049 |
| Axis coefficient (slope) [m] | -1.68 |
| Axis coefficient (intercept) [t] | 297.12 |

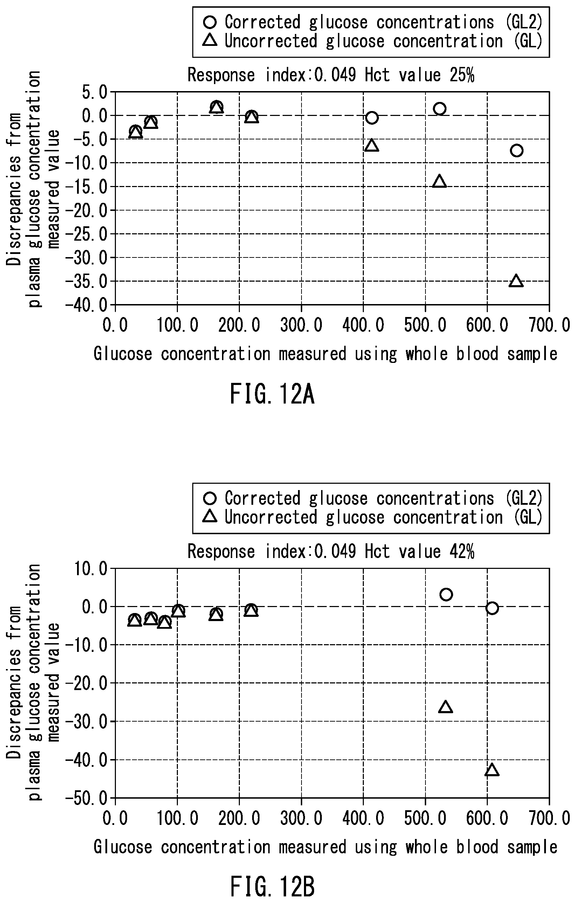

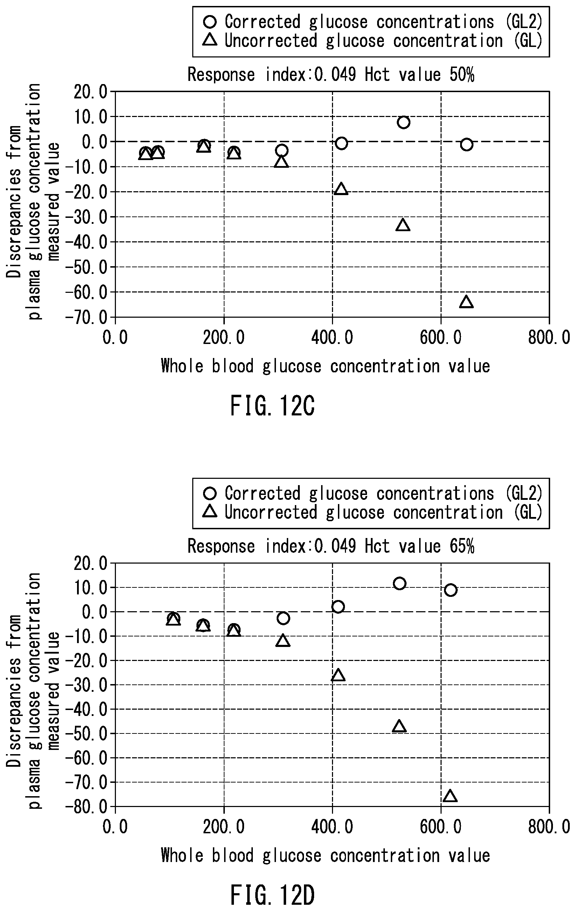

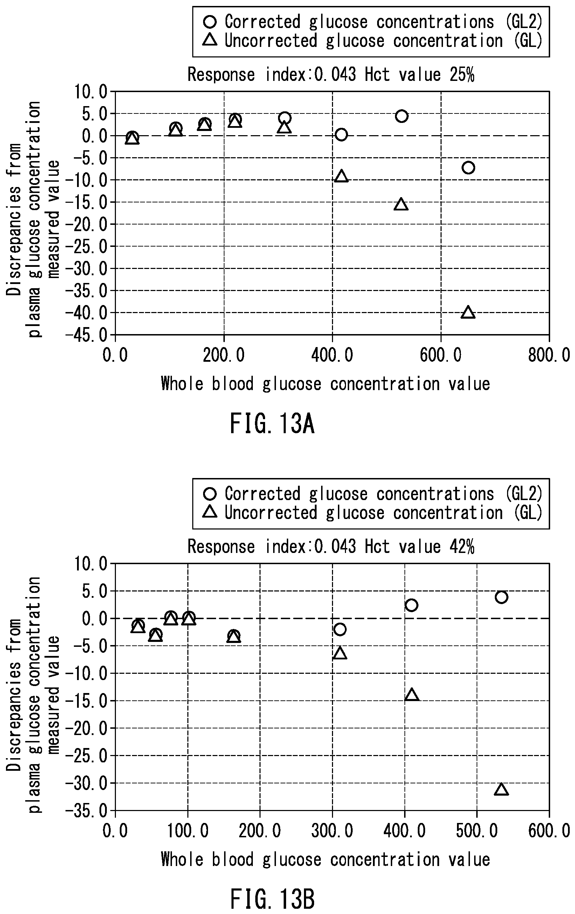

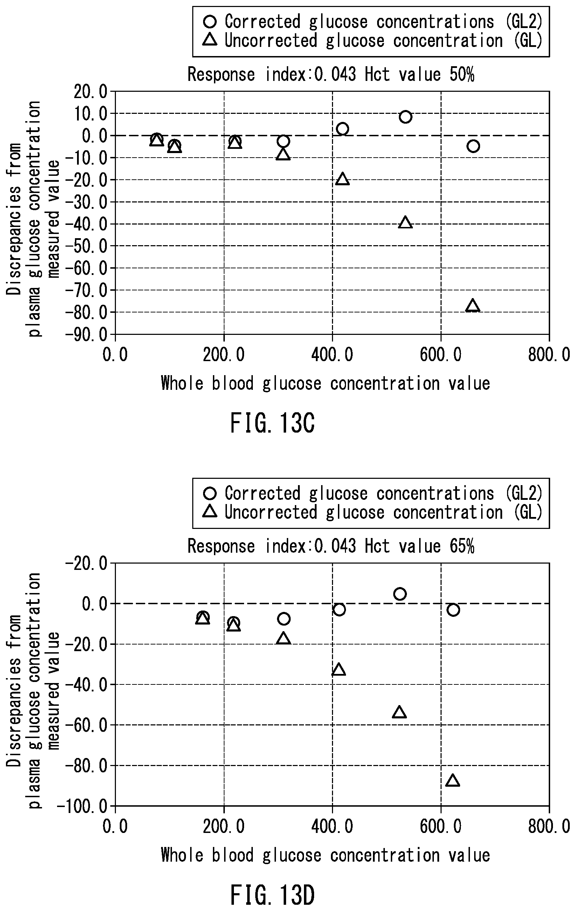

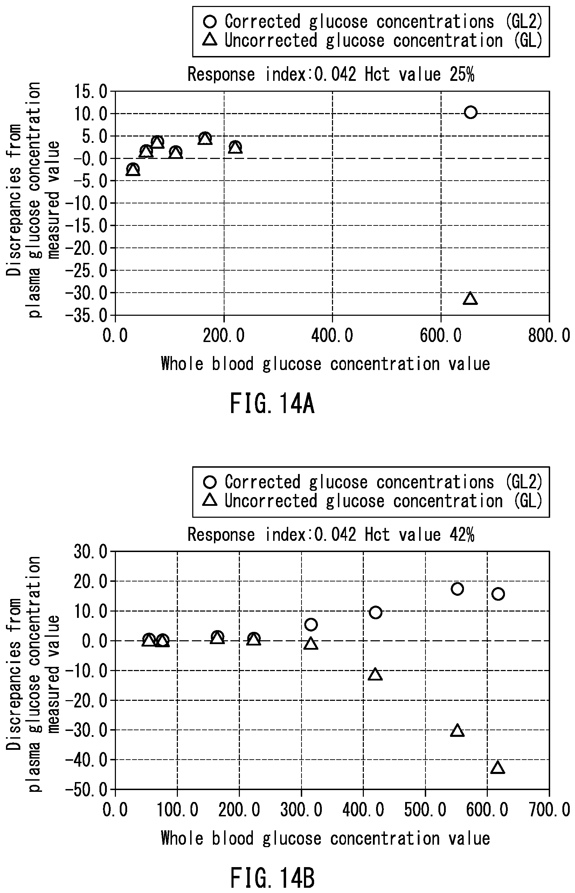

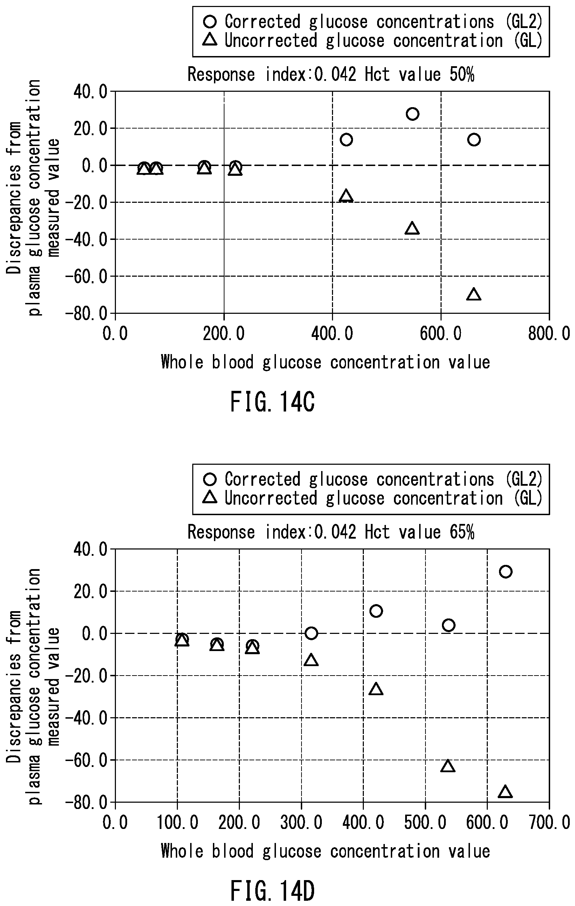

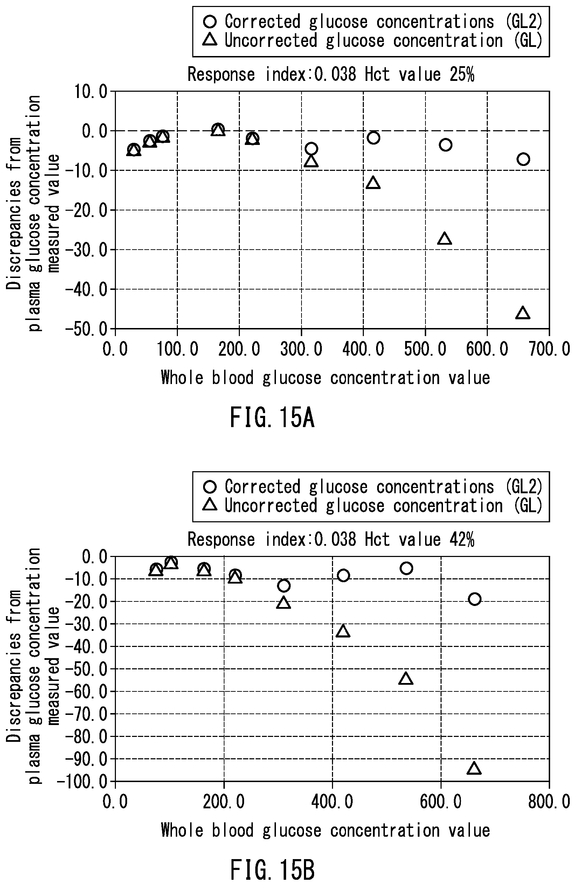

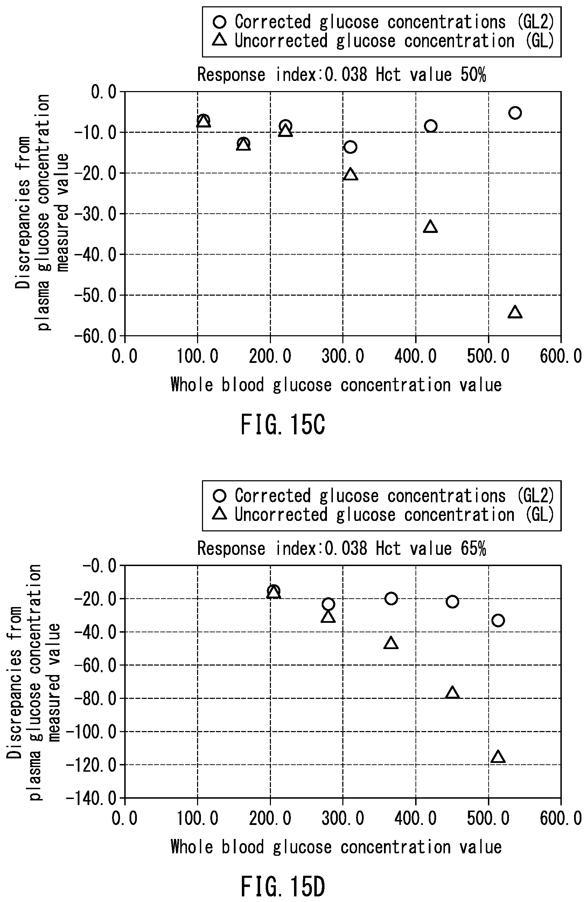

FIGS. 12A to 15D show the results. FIGS. 15A to 15D, FIGS. 14A to 14D, FIGS. 13A to 13D, and FIGS. 12A to 12D show discrepancies of the glucose concentration measured values (GL) and the corrected glucose concentration values (plasma glucose concentrations) (GL2) from glucose concentration measured values measured from plasma, at response indexes of 0.038, 0.042, 0.043, and 0.049, respectively.

As shown in FIGS. 12A to 15D, it can be seen that the discrepancies between the glucose concentration values (plasma glucose concentrations) (GL2) corrected using the method of the present disclosure and the glucose concentration measured values measured from plasma are smaller than those of the glucose concentration measured values (GL) obtained using the method disclosed in JP H9-318634A.

The present disclosure further relates to one or more non-limiting embodiments below.

[A1] A method for measuring a component concentration in blood plasma using a whole blood sample, the method comprising:

measuring a component concentration in the whole blood sample using the whole blood sample, using an electrochemical sensor method;
computing a correction value using the component concentration in the whole blood sample, a hematocrit value of the whole blood sample, and a response index of a sensor used for the measurement; and
computing the component concentration in blood plasma using the correction value and the component concentration in the whole blood sample.

[A2] The method according to [A1], comprising:

computing the correction value using a formula (a) below; and

$$\text{Correction value} = Hct \times p \times (r/r1) \times [CW - \{(Hct \times m + n) \times (r1/r)\}]^2 \cdots (a)$$

wherein, in the formula (a),

Hct represents a hematocrit value of the whole blood sample,

17

CW represents the component concentration in the whole blood sample obtained through the measurement using the whole blood sample,

p represents correction value determination coefficient,

r represents standard response index,

r1 represents response index of the sensor used for the measurement of the component concentration in the whole blood sample,

m and n represent axis determination coefficient, and

p, r, m and n are constants.

correcting the component concentration in the whole blood sample to the component concentration in blood plasma using a formula (b) below:

$$CP = CW - \text{correction value} \cdots (b)$$

wherein, in the formula (b),

CP represents the component concentration in blood plasma,

CW represents the component concentration in the whole blood sample, and

Correction value is the correction value computed by the formula (a).

[A3] The method according to [A1] or [A2], wherein the component is glucose.

[A4] A method for measuring a glucose concentration in blood plasma using a whole blood sample, the method comprising:

obtaining, using a glucose sensor method and using a whole blood sample, a glucose concentration (EP) in the whole blood sample computed by an equilibrium point method and a glucose concentration (DI) in the whole blood sample computed by a first-order differentiation method;

obtaining a glucose concentration (GL) in the whole blood sample using a formula (1) below:

$$GL = EP + a \times (EP - DI) + b \cdots (1)$$

wherein, in the formula (1),

EP represents the glucose concentration in the whole blood sample computed by an equilibrium point method,

DI represents the glucose concentration in the whole blood sample computed by a first-order differentiation method,

a is a constant of $0.5 \leq a \leq 3.0$, and

b is a constant of $0 < b \leq 10$;

obtaining a correction value (CR) using a formula (9) below:

$$CR = Hct \times p \times (r/r1) \times [GL - \{(Hct \times m + n) \times (r1/r)\}]^2 \cdots (9)$$

wherein, in the formula (9),

Hct represents the hematocrit value of the whole blood sample,

GL represents the glucose concentration computed by the formula (1),

p represents correction value determination coefficient,

r represents standard response index,

r1 represents response index of the sensor used for the measurement of the component concentration in the whole blood sample,

m and n represent axis determination coefficient, and

p, r, m and n are constants, and

obtaining a glucose concentration (GL2) in blood plasma using a formula (10) below:

$$GL2 = GL - CR \cdots (10),$$

wherein, in the formula (10),

GL represents the glucose concentration computed by the formula (1),
CR represents the correction value computed by the formula (9).

[A5] The method according to [A3] or [A4], further comprising:

computing the hematocrit value (Hct value) of the whole blood sample using a formula (12) below:

$$Hct\ value = (EP - DI) \times \{s \times r1 + t\} \times \{(GL)^u\} \cdots (12)$$

wherein, in the formula (12),

EP represents the glucose concentration in the whole blood sample computed by the equilibrium point method,
DI represents the glucose concentration in the whole blood sample computed by the first-order differentiation method,
s, t and u represent Hct determination coefficient,
r1 represents response index of the sensor used for the measurement of the component concentration in the whole blood sample, and
GL represents the glucose concentration computed by the formula (1), and
s, t, and u are constants.

[A6] An apparatus for measuring a component concentration in blood plasma using a whole blood sample, comprising:

a measurement unit that measures a component concentration in the whole blood sample using the whole blood and using an electrochemical sensor method; and
a control unit that computes a component concentration in blood plasma from the component concentration in the whole blood sample obtained by the measurement unit,
wherein the control unit comprises:

computing a correction value using the component concentration obtained by the measurement unit, a hematocrit value of the whole blood sample, and a response index of a sensor used for the measurement by the measurement unit; and
computing a component concentration in blood plasma using the obtained correction value and the component concentration obtained by the measurement unit.

[A7] A non-transitory computer-readable storage medium storing a program for causing a computer to execute processing to determine a component concentration in blood plasma using a whole blood sample, the processing comprising:

a correction value computation step of computing a correction value using a measured value obtained by measuring a component concentration in the whole blood sample using an electrochemical sensor method, a hematocrit value of the whole blood sample, and a response index of a sensor used for the measurement of the component concentration in the whole blood sample; and
a concentration computation step of computing a component concentration in blood plasma using the correction value and the measured value.

[A8] A non-transitory computer-readable storage medium storing a program for causing a computer to execute processing to determine a plasma glucose concentration using a whole blood sample, the processing comprising:

a measurement step of measuring a glucose concentration in the whole blood sample using a glucose sensor method and obtaining a glucose concentration (EP) in the whole blood sample computed by an equilibrium point method and a glucose concentration (DI) in the whole blood sample computed by a first-order differentiation method;
a first concentration computation step of computing a glucose concentration (GL) in the whole blood sample using a formula (1) below:

$$GL = EP + a \times (EP - DI) + b \cdots (1)$$

wherein, in the formula (1),

EP represents the glucose concentration in the whole blood sample computed by the equilibrium point method,
DI represents the glucose concentration in the whole blood sample computed by the first-order differentiation method,
a is a constant of $0.5 \leq a \leq 3.0$, and
b is a constant of $0 < b \leq 10$;

a correction value computation step of computing a correction value (CR) using a formula (9) below:

$$CR = Hct \times p \times (r/r1) \times [GL - \{(Hct \times m + n) \times (r1/r)\}]^2 \cdots (9)$$

wherein, in the formula (9),

Hct represents a hematocrit value of the whole blood sample,
GL represents the glucose concentration computed by the formula (1),
p represents correction value determination coefficient,
r represents standard response index,
r1 represents response index of the sensor used for the measurement of the component concentration in the whole blood sample,
m and n represent axis determination coefficient, and
p, r, m and n are constants.

a second concentration computation step of computing a glucose concentration (GL2) in blood plasma using a formula (10) below:

$$GL2 = GL - CR \cdots (10)$$

wherein, in the formula (10),

GL represents the glucose concentration computed by the formula (1),
CR represents the correction value computed by the formula (9).

[A9] The non-transitory computer-readable storage medium storing the program according to [A8],
wherein the processing further comprises:

a hematocrit value computation step of computing the hematocrit value (Hct value) of the whole blood sample using a formula (12) below:

$$Hct\ value = (EP - DI) \times \{s \times r1 + t\} \times \{(GL)^u\} \cdots (12)$$

wherein, in the formula (12),

EP represents the glucose concentration in the whole blood sample computed by an equilibrium point method,
DI represents the glucose concentration in the whole blood sample computed by a first-order differentiation method,
s, t and u represent Hct determination coefficient,
r1 represents response index of the sensor used for the measurement of the component concentration in the whole blood sample, and
GL represents the glucose concentration computed by the formula (1), and
s, t, and u are constants.

[A10] A method for measuring a hematocrit value (Hct value) using a whole blood sample, the method comprising:

obtaining, using a glucose sensor method and using a whole blood sample, a glucose concentration (EP) in the whole blood sample computed by an equilibrium point method and a glucose concentration measured value (DI) in the whole blood sample computed by a first-order differentiation method;
obtaining a glucose concentration (GL) using a formula (1) below:

$$GL = EP + a \times (EP - DI) + b \cdots (1)$$

wherein, in the formula (1),

EP represents the glucose concentration in the whole blood sample computed by an equilibrium point method,
DI represents the glucose concentration in the whole blood sample computed by a first-order differentiation method,
a is a constant of $0.5 \leq a \leq 3.0$,
b is a constant of $0 < b \leq 10$; and

obtaining a hematocrit value (Hct value) using a formula (12) below:

$$Hct\ value = (EP - DI) \times \{s \times r1 + t\} \times \{(GL)^u\} \cdots (12)$$

wherein, in the formula (12),

EP represents the glucose concentration in the whole blood sample computed by an equilibrium point method,
DI represents the glucose concentration in the whole blood sample computed by a first-order differentiation method,
s, t and u represent Hct determination coefficient,
r1 represents response index of the sensor used for the measurement of the component concentration in the whole blood sample, and
GL represents the glucose concentration computed by the formula (1), and
s, t, and u are constants.

[A11] A non-transitory computer-readable storage medium storing a program for causing a computer to execute processing to determine a hematocrit value (Hct value) using a whole blood sample, the processing comprising:

a measurement step of measuring a glucose concentration in the whole blood sample using a glucose sensor method and obtaining a glucose concentration (EP) in the whole blood sample computed by an equilibrium point method and a glucose concentration (DI) in the whole blood sample computed by a first-order differentiation method;
a concentration computation step of computing a glucose concentration (GL) in the whole blood sample using a formula (1) below:

$$GL = EP + a \times (EP - DI) + b \cdots (1)$$

wherein, in the formula (1),

EP represents the glucose concentration in the whole blood sample computed by an equilibrium point method,
DI represents the glucose concentration in the whole blood sample computed by a first-order differentiation method,
a is a constant of $0.5 \leq a \leq 3.0$,

b is a constant of $0 < b \leq 10$; and

a hematocrit value computation step of computing a hematocrit value (Hct value) using a formula (12) below:

$$\text{Hct value} = (EP - DI) \times \{s \times r1 + t\} \times \{(GL)^u\} \cdots (12)$$

wherein, in the formula (12),

EP represents the glucose concentration in the whole blood sample computed by an equilibrium point method,
DI represents the glucose concentration in the whole blood sample computed by a first-order differentiation method,
s, t and u represent Hct determination coefficient,
r1 represents response index of the sensor used for the measurement of the component concentration in the whole blood sample, and
GL represents the glucose concentration computed by the formula (1), and
s, t, and u are constants.

[B1] A method for measuring a component concentration in a whole blood sample, the method including: correcting a measured value from a whole blood sample to a measured value from a plasma sample by performing correction using a correction formula that includes the multiplication of a term related to a hematocrit value of a sample by a term related to a response index of a sensor.

[B2] The method for measuring a component concentration in a whole blood sample according to [B1], wherein the measured value from the plasma sample is used as a corrected component concentration, and the whole blood component concentration measured value is corrected to the corrected component concentration using a formula below:

Corrected component concentration = whole blood component concentration measured value - correction value Correction value = hematocrit value $\times$ correction value determination coefficient $\times$ (standard response index / response index) $\times$ [whole blood component concentration measured value - {(hematocrit value $\times$ axis determination coefficient + axis determination coefficient) $\times$ (response index / standard response index)}]$^2$

With each of the above-disclosed methods, the method is preferably a computer-implemented method in which at least the computation or calculation steps are performed by a computer or other processor running a program which causes the computation or calculation steps to be executed.

The present invention may be embodied in other forms without departing from the spirit or essential characteristics thereof. The embodiments disclosed in this application are to be considered in all respects as illustrative and not limiting. The scope of the present invention is indicated by the appended claims rather than by the foregoing description, and all changes which come within the meaning and range of equivalency of the claims are intended to be embraced therein.

**Claims**

1.  A method for measuring a plasma component concentration using a whole blood sample, the method comprising:

    measuring a whole blood component concentration using a whole blood sample, using an electrochemical sensor method;
    computing a correction value using a whole blood component concentration measured value obtained through the measurement, a hematocrit value of the whole blood sample, and a response index of a sensor used for the measurement of the whole blood component concentration; and
    computing a plasma component concentration using the correction value and the whole blood component concentration measured value.

2.  The method according to claim 1, comprising:

computing the correction value using a formula (a) below; and
correcting the whole blood component concentration measured value to the plasma component concentration using the computed correction value and using a formula (b) below:

Correction value = hematocrit value × correction value determination coefficient p × (standard response index r / response index) × [whole blood component concentration measured value - {(hematocrit value × axis determination coefficient m + axis determination coefficient n) × (response index / standard response index r)}]$^2$ (a)

Plasma component concentration = whole blood component concentration measured value - correction value (b)

wherein, in the formula (a), the hematocrit value is the hematocrit value of the whole blood sample, the response index is the response index of the sensor used for the measurement of the whole blood component concentration, and the correction value determination coefficient p, the standard response index r, and the axis determination coefficients m and n are constants.

3. The method according to claim 1 or 2, wherein the component is glucose.

4. A method for measuring a plasma glucose concentration using a whole blood sample, the method comprising:

obtaining a glucose concentration measured value (EP) computed by an equilibrium point method and a glucose concentration measured value (DI) computed by a first-order differentiation method, using a glucose sensor method and using a whole blood sample;
obtaining a whole blood glucose concentration value (GL) using the obtained glucose concentration measured value (EP) and glucose concentration measured value (DI), using a formula (1) below:

$$GL = EP + a \times (EP - DI) + b \cdots (1)$$

wherein, in the formula (1), a is a constant of $0.5 \leq a \leq 3.0$, and b is a constant of $0 < b \leq 10$;
obtaining a correction value (CR) using the whole blood glucose concentration value (GL), a hematocrit value (Hct value) of the whole blood sample, a response index of a sensor used for the glucose concentration measurements using the glucose sensor method, and a standard response index r, using a formula (9) below:

CR = Hct value × correction value determination coefficient p × (standard response index r / response index) × [GL - {(Hct value × axis determination coefficient m + axis determination coefficient n) × (response index / standard response index r)}]$^2$ (9)

wherein, in the formula (9), the correction value determination coefficient p, the standard response index r, and the axis determination coefficients m and n are constants; and
obtaining a plasma glucose concentration (GL2) using the whole blood glucose concentration value (GL) and the correction value (CR), using a formula (10) below:

$$GL2 = GL - CR \cdots (10).$$

5. The method according to claim 3 or 4, further comprising:

computing the hematocrit value (Hct value) of the whole blood sample using a formula (12) below:

Hematocrit value = (EP - DI) × {[Hct determination coefficient s] × response index + [Hct determination coefficient t]} × {(GL)$^{[Hct\ determination\ coefficient\ u]}$} (12)

wherein, in the formula (12), the Hct determination coefficients s, t, and u are constants.

6. An apparatus for measuring a plasma component concentration using a whole blood sample, comprising:

a measurement unit configured to measure a whole blood component concentration in a whole blood sample using an electrochemical sensor method; and

a control unit configured to calculate a plasma component concentration from a measured value obtained by the measurement unit,

wherein the control unit is configured:

to calculate a correction value using the whole blood component concentration measured value obtained by the measurement unit, a hematocrit value of the whole blood sample, and a response index of a sensor used for the measurement of the whole blood component concentration by the measurement unit; and

to calculate a plasma component concentration using the obtained correction value and the whole blood component concentration measured value.

7. A non-transitory computer-readable storage medium storing a program for causing a computer to execute processing to determine a plasma component concentration using a whole blood sample, the processing comprising:

a correction value computation step of computing a correction value using a measured value obtained by measuring a whole blood component concentration in the whole blood sample using an electrochemical sensor method, a hematocrit value of the whole blood sample, and a response index of a sensor used for the measurement of the whole blood component concentration; and

a concentration computation step of computing a plasma component concentration using the correction value and the measured value.

8. A non-transitory computer-readable storage medium storing a program for causing a computer to execute processing to determine a plasma glucose concentration using a whole blood sample, the processing comprising:

a measurement step of performing, using a glucose sensor method and using a whole blood sample, glucose concentration measurements using an equilibrium point method and a first-order differentiation method;

a first concentration computation step of computing a whole blood glucose concentration value (GL) using a glucose concentration measured value (EP) computed by the equilibrium point method and a glucose concentration measured value (DI) computed by the first-order differentiation method, respectively, in the measurement step, using a formula (1) below:

$$GL = EP + a \times (EP - DI) + b \cdots (1)$$

wherein, in the formula (1), a is a constant of $0.5 \leq a \leq 3.0$, and b is a constant of $0 < b \leq 10$;

a correction value computation step of computing a correction value (CR) using the whole blood glucose concentration value (GL) obtained in the first concentration computation step, a hematocrit value (Hct value) of the whole blood sample, a response index of a sensor used for the glucose concentration measurements, and a standard response index r, using a formula (9) below:

CR = Hct value $\times$ correction value determination coefficient p $\times$ (standard response index r / response index) $\times$ [GL - {(Hct value $\times$ axis determination coefficient m + axis determination coefficient n) $\times$ (response index / standard response index r)}]$^2$    (9)

wherein, in the formula (9), the correction value determination coefficient p, the standard response index r, and the axis determination coefficients m and n are constants; and

a second concentration computation step of computing a plasma glucose concentration (GL2) using the whole blood glucose concentration value (GL) and the correction value (CR), using a formula (10) below:

$$GL2 = GL - CR \cdots (10).$$

9. The non-transitory computer-readable storage medium storing the program according to claim 8, wherein the processing further comprises:

a hematocrit value computation step of computing the hematocrit value (Hct value) of the whole blood sample using a formula (12) below:

Hematocrit value = (EP - DI) × {[Hct determination coefficient s] × response index + [Hct determination coefficient t]} × {(GL)$^{[Hct\ determination\ coefficient\ u]}$} (12)

wherein, in the formula (12), the Hct determination coefficients s, t, and u are constants.

10. A method for measuring a hematocrit value (Hct value) using a whole blood sample, the method comprising:

obtaining, using a glucose sensor method and using a whole blood sample, a glucose concentration measured value (EP) computed by an equilibrium point method and a glucose concentration measured value (DI) computed by a first-order differentiation method;
obtaining a whole blood glucose concentration value (GL) using the glucose concentration measured value (EP) obtained by the equilibrium point method and the glucose concentration measured value (DI) obtained by the first-order differentiation method, using a formula (1) below:

$$GL = EP + a × (EP − DI) + b \cdots (1)$$

wherein, in the formula (1), a is a constant of $0.5 \leq a \leq 3.0$, and b is a constant of $0 < b \leq 10$; and
obtaining a hematocrit value (Hct value) using the glucose concentration measured value (EP), the glucose concentration measured value (DI), and the whole blood glucose concentration value (GL), using a formula (12) below:

Hematocrit value = (EP - DI) × {[Hct determination coefficient s] × response index + [Hct determination coefficient t]} × {(GL)$^{[Hct\ determination\ coefficient\ u]}$} (12)

wherein, in the formula (12), the Hct determination coefficients s, t, and u are constants.

11. A non-transitory computer-readable storage medium storing a program for causing a computer to execute processing to determine a hematocrit value (Hct value) using a whole blood sample, the processing comprising:

a measurement step of performing, using a glucose sensor method and using a whole blood sample, glucose concentration measurements using an equilibrium point method and a first-order differentiation method;
a concentration computation step of computing a whole blood glucose concentration value (GL) using a glucose concentration measured value (EP) and a glucose concentration measured value (DI) obtained by the equilibrium point method and the first-order differentiation method, respectively, in the measurement step, using a formula (1) below:

$$GL = EP + a × (EP − DI) + b \cdots (1)$$

wherein, in the formula (1), a is a constant of $0.5 \leq a \leq 3.0$, and b is a constant of $0 < b \leq 10$; and
a hematocrit value computation step of computing a hematocrit value (Hct value) using the glucose concentration measured value (EP), the glucose concentration measured value (DI), and the whole blood glucose concentration value (GL), using a formula (12) below:

Hematocrit value = (EP - DI) × {[Hct determination coefficient s] × response index+ [Hct determination coefficient t]} × {(GL)$^{[Hct\ determination\ coefficient\ u]}$} (12)

wherein, in the formula (12), the Hct determination coefficients s, t, and u are constants.

FIG. 1

FIG. 2

FIG. 3

FIG. 4

FIG. 5

FIG. 6

FIG. 7

```
                    ┌─────────────────┐
                    │      START      │
                    └─────────────────┘
                             │
                             ▼
          ┌──────────────────────────────────┐
          │  Introduction of buffer solution │────S801
          │        into reaction cell 21     │
          └──────────────────────────────────┘
                             │
                             ▼
          ┌──────────────────────────────────┐
          │  Introduction of whole blood sample│──S802
          │        into reaction cell 21     │
          └──────────────────────────────────┘
                             │
                             ▼
          ┌──────────────────────────────────┐
          │    Start measuring a glucose     │────S803
          │ concentration in whole blood sample│
          └──────────────────────────────────┘
                             │
                             ▼
          ┌──────────────────────────────────┐
          │    Computing whole blood glucose │────S804
          │           concentration          │
          └──────────────────────────────────┘
                             │
                             ▼
          ┌──────────────────────────────────┐
          │     Computing correction value   │────S805
          └──────────────────────────────────┘
                             │
                             ▼
          ┌──────────────────────────────────┐
          │      Computing plasma glucose    │────S806
          │           concentration          │
          └──────────────────────────────────┘
                             │
                             ▼
                    ┌─────────────────┐
                    │       END       │
                    └─────────────────┘
```

FIG. 8

START

Introduction of buffer solution into reaction cell 21 ——S901

Introduction of whole blood sample into reaction cell 21 ——S902

Start measuring a glucose concentration in whole blood sample ——S903

_S904 Obtaining a glucose concentration (EP) computed by an equilibrium point method

_S905 Obtaining a glucose concentration (DI) computed by a first-order differentiation method

Computing whole blood glucose concentration value (GL) ——S906

Computing correction value (CR) ——S907

Computing plasma glucose concentration (GL2) ——S908

END

FIG. 9

START

Introduction of buffer solution into reaction cell 21 — S1001

Introduction of whole blood sample into reaction cell 21 — S1002

Start measuring a glucose concentration in whole blood sample — S1003

— S1004
Obtaining a glucose concentration (EP) computed by an equilibrium point method

— S1005
Obtaining a glucose concentration (DI) computed by a first-order differentiation method

Computing whole blood glucose concentration value (GL) — S1006

Computing Hematocrit value — S1007

Computing correction value (CR) — S1008

Computing plasma glucose concentration (GL2) — S1009

END

FIG. 10

```
                            ┌──────────────┐
                            │    START     │
                            └──────┬───────┘
                                   │
                                   ▼
              ┌────────────────────────────────────┐
              │  Introduction of buffer solution   │──── S1101
              │        into reaction cell 21       │
              └────────────────────┬───────────────┘
                                   │
                                   ▼
              ┌────────────────────────────────────┐
              │  Introduction of whole blood sample │──── S1102
              │        into reaction cell 21        │
              └────────────────────┬───────────────┘
                                   │
                                   ▼
              ┌────────────────────────────────────┐
              │       Start measuring a glucose     │──── S1103
              │   concentration in whole blood sample│
              └────────────────────┬───────────────┘
                                   │
              ┌────────────────────┴────────────────────┐
              │ S1104                          S1105     │
              ▼                                 ▼
  ┌──────────────────────────────┐  ┌──────────────────────────────┐
  │ Obtaining a glucose concentration│  │ Obtaining a glucose concentration│
  │ (EP) computed by an equilibrium  │  │ (DI) computed by a first-order   │
  │        point method              │  │     differentiation method       │
  └──────────────┬───────────────┘  └───────────────┬──────────────┘
                 └───────────────────┬───────────────┘
                                     ▼
              ┌────────────────────────────────────┐
              │     Computing whole blood glucose    │──── S1106
              │        concentration value (GL)      │
              └────────────────────┬───────────────┘
                                   │
                                   ▼
              ┌────────────────────────────────────┐
              │       Computing Hematocrit value     │──── S1107
              └────────────────────┬───────────────┘
                                   │
                                   ▼
                            ┌──────────────┐
                            │     END      │
                            └──────────────┘
```

FIG. 11

FIG. 12A

FIG. 12B

○ Corrected glucose concentrations (GL2)
△ Uncorrected glucose concentration (GL)

Response index:0.049 Hct value 50%

FIG. 12C

○ Corrected glucose concentrations (GL2)
△ Uncorrected glucose concentration (GL)

Response index:0.049 Hct value 65%

FIG. 12D

FIG. 13A

FIG. 13B

FIG. 13C

FIG. 13D

FIG. 14A

FIG. 14B

FIG. 14C

FIG. 14D

FIG. 15A

FIG. 15B

○ Corrected glucose concentrations (GL2)
△ Uncorrected glucose concentration (GL)

Response index:0.038 Hct value 50%

FIG. 15C

○ Corrected glucose concentrations (GL2)
△ Uncorrected glucose concentration (GL)

Response index:0.038 Hct value 65%

FIG. 15D

**EP 4 679 074 A1**

Europäisches Patentamt
European Patent Office
Office européen des brevets

# EUROPEAN SEARCH REPORT

**Application Number**

EP 25 18 8159

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP H09 318634 A (KYOTO DAIICHI KAGAKU KK) 12 December 1997 (1997-12-12) * paragraphs [0001], [0002], [0006] - [0015], [0025] - [0030] * | 1,3,6,7 | INV. G01N27/327 G01N33/49 |
| X | JP 2005 148058 A (ARKRAY INC) 9 June 2005 (2005-06-09) * paragraphs [0001], [0004] - [0009], [0024] - [0053]; claims 1-17; figures 1-2 * | 1,3,6,7 | |
| X | JP H09 33533 A (KYOTO DAIICHI KAGAKU KK) 7 February 1997 (1997-02-07) * paragraphs [0027] - [0037]; claims 1-5 * | 1,3,6,7 | |
| A | WO 2006/046538 A1 (ARKRAY INC [JP]; SUGIYAMA KOJI [JP]; TAKAGI TAKESHI [JP]) 4 May 2006 (2006-05-04) * the whole document * | 1-11 | |
| A | EP 1 742 045 B1 (PANASONIC HEALTHCARE HOLDINGS CO LTD [JP]) 2 November 2016 (2016-11-02) * example 2 * | 1-11 | **TECHNICAL FIELDS SEARCHED (IPC)** G01N |

The present search report has been drawn up for all claims

| Place of search | Date of completion of the search | Examiner |
|---|---|---|
| Munich | 12 November 2025 | Lazar, Zala |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

43

Europäisches
Patentamt

European
Patent Office

Office européen
des brevets

**Application Number**

EP 25 18 8159

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

Application Number

**EP 25 18 8159**

The Search Division considers that the present European patent application does not comply with the requirements of unity of invention and relates to several inventions or groups of inventions, namely:

```
    1. claims: 1-11

        A method, an apparatus and a computer program for
        determining glucose concentration in blood plasma

    1.1. claims: 10, 11

        A method and a computer program for determining a hematocrit
        value in a whole blood sample
                            ---
```

```
Please note that all inventions mentioned under item 1, although not
necessarily linked by a common inventive concept, could be searched
without effort justifying an additional fee.
```

## ANNEX TO THE EUROPEAN SEARCH REPORT
## ON EUROPEAN PATENT APPLICATION NO.

EP 25 18 8159

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2025

| Patent document cited in search report | | Publication date | Patent family member(s) | | Publication date |
|---|---|---|---|---|---|
| JP H09318634 | A | 12-12-1997 | JP | 3698281 B2 | 21-09-2005 |
| | | | JP | H09318634 A | 12-12-1997 |
| JP 2005148058 | A | 09-06-2005 | JP | 4576505 B2 | 10-11-2010 |
| | | | JP | 2005148058 A | 09-06-2005 |
| JP H0933533 | A | 07-02-1997 | JP | 3700140 B2 | 28-09-2005 |
| | | | JP | H0933533 A | 07-02-1997 |
| WO 2006046538 | A1 | 04-05-2006 | CN | 101048657 A | 03-10-2007 |
| | | | EP | 1806580 A1 | 11-07-2007 |
| | | | JP | 4660652 B2 | 30-03-2011 |
| | | | JP | WO2006046538 A1 | 22-05-2008 |
| | | | TW | I286206 B | 01-09-2007 |
| | | | US | 2008118942 A1 | 22-05-2008 |
| | | | WO | 2006046538 A1 | 04-05-2006 |
| EP 1742045 | B1 | 02-11-2016 | CA | 2559297 A1 | 03-11-2005 |
| | | | CN | 1938590 A | 28-03-2007 |
| | | | EP | 1742045 A1 | 10-01-2007 |
| | | | EP | 3115777 A1 | 11-01-2017 |
| | | | JP | 4689601 B2 | 25-05-2011 |
| | | | JP | 4696183 B2 | 08-06-2011 |
| | | | JP | 4751479 B2 | 17-08-2011 |
| | | | JP | 5502015 B2 | 28-05-2014 |
| | | | JP | 5619665 B2 | 05-11-2014 |
| | | | JP | 2011033637 A | 17-02-2011 |
| | | | JP | 2011033638 A | 17-02-2011 |
| | | | JP | 2011137839 A | 14-07-2011 |
| | | | JP | 2011158483 A | 18-08-2011 |
| | | | JP | WO2005103669 A1 | 13-03-2008 |
| | | | KR | 20060134101 A | 27-12-2006 |
| | | | US | 2007138026 A1 | 21-06-2007 |
| | | | US | 2011198223 A1 | 18-08-2011 |
| | | | US | 2014158552 A1 | 12-06-2014 |
| | | | US | 2016178557 A1 | 23-06-2016 |
| | | | US | 2018120249 A1 | 03-05-2018 |
| | | | WO | 2005103669 A1 | 03-11-2005 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H9318634 A

- JP 2004170401 A